# EUROPEAN PATENT SPECIFICATION

(11) **EP 4 114 256 B1**
(45) Date of publication and mention of the grant of the patent: **13.08.2025**
(21) Application number: 21716894.7
(22) Date of filing: 11.03.2021
(51) Int. Cl.: A61B 5/0215, F16K 1/54

(54) **PRESSURE TRANSDUCER COMPRISING POPPET VALVE, AND METHOD**
DRUCKWANDLER MIT TELLERVENTIL, UND VERFAHREN
TRANSDUCTEUR DE PRESSION COMPRENANT UNE SOUPAPE À CLAPET, ET PROCÉDÉ

(30) Priority: 24.03.2020 US 202062994102 P
(43) Date of publication of application: 11.01.2023
(73) Proprietor: Becton, Dickinson and Company, Franklin Lakes, NJ 07417-1880 (US)
(72) Inventor: KHORANA, Christopher Arjun, Irvine, CA 92614 (US); HARPS, Daniel M., Irvine, CA 92614 (US); HU, Weiwei, Irvine, CA 92614 (US); REY, Jonathan Pierre, Irvine, CA 92614 (US); LUTGEN, Joseph Leon, Costa Mesa, CA 92626 (US)
(74) Representative: Venner, Julia Ann
(86) International application number: PCT/US2021/021992
(87) International publication number: WO 2021/194762

(56) References cited:
- EP-A1- 0 331 526
- JP-A- 2007 085 223
- US-A- 4 947 856
- US-A1- 2009 194 174

## Description

### FIELD OF THE INVENTION

The present invention relates to pressure transducers and, more particularly, the present invention is directed to a pressure transducer for monitoring and recording hemodynamic pressures within an individual, and a method of flushing a pressure transducer.

### BACKGROUND

When diagnosing and treating various bodily ailments, such as shock or cardiovascular problems, medical personnel often find it desirable to measure and/or monitor a patient's blood pressure. By monitoring the blood pressure medical personnel are better able to detect blood flow difficulties and other cardiovascular problems at an early stage. As a result, the use of blood pressure measurement and monitoring devices can increase the likelihood that a patient be successfully treated or provided with needed emergency assistance.

A variety of methods are currently used for measuring and monitoring blood pressure. For example, medical personnel frequently use various indirect blood pressure measurement techniques, such as measuring a patient's blood pressure by using a pressure cuff and a stethoscope. In addition, blood pressure measurements are often made using a number of direct measurement and monitoring techniques. Notably, when diagnosing and treating critically ill patients, such direct techniques are generally preferred over any of the indirect techniques. Direct blood pressure measurement and monitoring techniques are generally accurate to within about one percent, and facilitate the continuous monitoring of a patient's blood pressure on a beat-to-beat basis. Direct blood pressure monitoring also enables the rapid detection of a change in cardiovascular activity, which may be of significant importance in emergency situations. The direct measurement method has been more widely used than the indirect measurement method with respect to a patient who is being treated in an operating room or intensive care unit. This is because blood pressure can be measured at the same time as execution of blood operations such as sampling of blood and injection of medicine. Furthermore, high-precision measurement of the blood pressure can be realized and long-time continuous monitoring can be enabled.

In direct blood pressure monitoring systems, a catheter is inserted into a patient's circulatory system with the end of the catheter having an opening to the blood stream, typically in a major or peripheral blood vessel. An I.V. set attaches to the proximal end of the catheter protruding from the patient so that a solution flows through the catheter and into the patient. The I.V. solution provides a fluid "column" through which pressure pulses are transmitted, and a pressure transducer positioned along the fluid column monitors those pressure pulses.

In the past, the pressure transducer consisted of a dome that functions as a reservoir for the I.V. fluid. The dome includes a resilient diaphragm that attaches to an electrical transducer. The transducer senses pressure fluctuations in the diaphragm and converts them into electrical signals which then transmit through a cable to a monitor for amplification and display. In modern systems a single silicon chip comprises both the pressure diaphragm and the measuring circuitry of the pressure transducer. Since such silicon chips are cheaply mass-produced, the total cost of pressure transducers is reduced to the extent that the transducer becomes economically disposable. Such disposable blood pressure transducers (DPTs) are the standard of care in the OR, ICU, or CCU.

EP 0 331 526 describes an improved flush-valve assembly for a blood pressure measurement catheter. A resilient valve core, biased against its seat in a molded body, controls large volumes for flushing. A small resilient tube embedded in the core bypasses the valve to provide smaller flow volumes for IV-fluid drip.

### SUMMARY OF THE INVENTION

Aspects of the presently claimed invention are set out in the independent claims. Particular embodiments of these aspects are set out in the dependent claims. Any subject matter contained herein that does not fall within the scope of the appended claims is considered as being useful for understanding the invention.

The present application discloses several pressure transducers and methods of assembling and using pressure transducers. In one example, a pressure transducer assembly directly monitors a pressure in a fluid that flows through the assembly. The pressure transducer can include a housing with an integral flow restrictor, an inlet port, and an outlet port.

In one example, a pressure transducer assembly includes a housing, a poppet, and a flow restrictor. The housing comprises a flow restrictor, an inlet port, and an outlet port. The poppet is coupled with the housing. The flow restrictor is defined by a valve seat between the inlet port and the outlet port.

In one exemplary method of flushing a pressure transducer, a fluid flows through a first flow path. The first flow path includes an inlet port, a flow restrictor, and an outlet port. The flow restrictor is disposed on a valve seat of a housing of the pressure transducer. A poppet is decoupled from the valve seat of the housing. This decoupling allows the fluid to
travel through a second flow path. The second flow path includes the inlet port, a by-pass channel, and the outlet port.

### BRIEF DESCRIPTION OF THE DRAWINGS

To further clarify various aspects of embodiments of the present disclosure, a more particular description of the certain embodiments will be made by reference to various aspects of the appended drawings. It is appreciated that these drawings depict only typical embodiments of the present disclosure and are therefore not to be considered limiting of the scope of the disclosure. Moreover, while the figures can be drawn to scale for some embodiments, the figures are not necessarily drawn to scale for all embodiments. Embodiments and other features and advantages of the present disclosure will be described and explained with additional specificity and detail through the use of the accompanying drawings in which:
FIG. 1 is an overhead view of a prior art disposable pressure transducer ("DPT");
FIG. 2 is a longitudinal sectional view of a housing of a prior art DPT;
FIG. 3 is a cross-sectional view of a housing of an exemplary embodiment of a DPT;
FIG. 4 is a cross-sectional view of a housing and a poppet of a DPT in an engaged state;
FIG. 5 is a cross-sectional view of a housing and a poppet of a DPT in a disengaged state;
FIG. 6 a cross-sectional view of a housing and a poppet of a DPT in a disengaged state:
FIG. 7 is a top perspective view of a DPT with a poppet removed;
FIG. 8 schematically illustrates flow when a poppet of a DPT is in the engaged state;
FIG. 9A is a cross-sectional view of a DPT having an exemplary overpressure feature;
FIG. 9B is a cross-sectional view of a DPT having an exemplary overpressure feature;
FIG. 10A schematically illustrates flow of the FIG. 9A embodiment when a poppet of a DPT is in a disengaged state;
FIG. 10B schematically illustrates flow of the FIG. 9B when a poppet of a DPT is in a disengaged state;
FIG. 11 is a top view of an alternate flow restrictor of a DPT;
FIGS. 12A-12E are cross sectional views of mold projections used to make flow restrictor channels for a DPT;
FIG. 13 illustrates a graphical relationship between the flow rate and pre-load of a flow restrictor channel of a DPT;
FIG. 14A-14E are cross sectional views of mold projections used to make flow restrictor channels for a DPT;
FIG. 15 illustrates a graphical relationship between the flow rate and pre-load of a flow restrictor channel of a DPT;
FIG. 16 is an enlarged perspective view of a port of a DPT;
FIG. 17 is a perspective view of a mold insert for making a DPT;
FIG. 18 is a perspective view of a housing and a poppet of a DPT;
FIG. 19A is a perspective view of a poppet of a DPT;
FIG. 19B is a cross sectional view of a poppet of a DPT;
FIG. 20 is a cross sectional view of an exemplary embodiment of a housing and a poppet of a DPT in a disassembled state;
FIG. 21 is a cross sectional view of an exemplary embodiment of a housing being assembled with a poppet;
FIGS. 22A and 22B schematically illustrate securing a poppet to a housing of an exemplary embodiment of a DPT;
FIG. 23 is a cross-sectional view of an exemplary embodiment of an assembled poppet and housing of a DPT;
FIG. 24 is a perspective view of an exemplary embodiment of a DPT;
FIG. 25 is a perspective view of an exemplary embodiment of a DPT;
FIG. 26 is a perspective view of an exemplary embodiment of an assembly of a housing and a cable of a DPT;
FIG. 26A is a cross-sectional view taken along the plane indicated by lines 26-26 in FIG. 26;
FIG. 26B is a schematic illustration of a wire end anchoring configuration taken from the perspective of the cross-section taken along the plane indicated by lines 26-26 in FIG. 26;
FIG. 26C is a schematic illustration of a wire end anchoring configuration taken from the perspective of the cross-section taken along the plane indicated by lines 26-26 in FIG. 26;
FIG. 26D is a schematic illustration of a wire end anchoring configuration taken from the perspective of the cross-section taken along the plane indicated by lines 26-26 in FIG. 26;
FIG. 27 is a perspective view of an exemplary embodiment of a sensor assembly and a housing of a DPT;
FIG. 28 is a perspective view of an exemplary embodiment of a pressure sensor assembly and a cable of a DPT;
FIG. 29 is a cross sectional view of the DPT of Figure 25 taken along the plane indicated by lines E-E in FIG. 25;
FIG. 30 is a cross sectional view of the DPT of Figure 29 taken along the plane indicated by lines F-F in FIG. 29;
FIG. 31 is a cross sectional view of the DPT of Figure 29 taken along the plane indicated by lines G-G in FIG. 29;
FIG. 32 is a cross sectional view of the DPT of Figure 29 taken along the plane indicated by lines H-H in FIG. 29;
FIG. 33 is a cross sectional view of the DPT of Figure 29 taken along the plane indicated by lines I-I in FIG. 29; and
FIG. 34 is a cross sectional view of the DPT of Figure 29 taken along the plane indicated by lines J-J in FIG. 29.

### DETAILED DESCRIPTION

The following description refers to the accompanying drawings, which illustrate specific embodiments of the present disclosure. Other embodiments having different structures and operation do not depart from the scope of the present disclosure.

Exemplary embodiments of the present disclosure are directed to devices and methods for remodeling the shape of one or more walls of a human heart. It should be noted that various embodiments of devices and systems for delivery are disclosed herein, and any combination of these options can be made unless specifically excluded. In other words, individual components of the disclosed devices and systems can be combined unless mutually exclusive or otherwise physically impossible.

As described herein, when one or more components are described as being connected, joined, affixed, coupled, attached, or otherwise interconnected, such interconnection may be direct as between the components or may be indirect such as through the use of one or more intermediary components. Also, as described herein, reference to a "member," "component," or "portion" shall not be limited to a single structural member, component, or element but can include an assembly of components, members, or elements. Also, as described herein, the terms "substantially" and "about" are defined as at least close to (and includes) a given value or state (preferably within 10% of, more preferably within 1% of, and most preferably within 0.1% of).

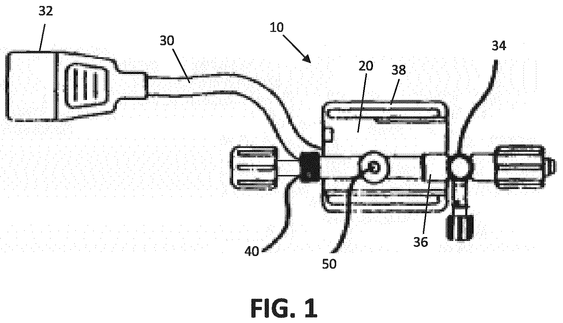

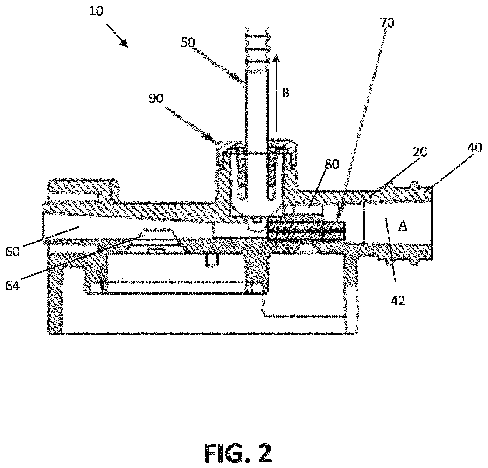

With reference to FIGs. 1 and 2, various components of a prior art disposable blood pressure transducing device (DPT) 10 are illustrated. The DPT 10 includes a housing 20, a cable 30 extending from one end of the housing 20 and terminating in an electrical connector 32, and a multi-port stopcock assembly 34. Although not shown in these figures, an internal flow channel in the stopcock assembly 34 leads to a short length of tubing 36 situated on a top side of the housing 20 opposite a mounting plate 38. The mounting plate 38 can engage walls in the mounting bracket (not shown), such that the tubing 36 faces outward from the mounting bracket. The DPT 10 can be connected to an external sterilized-liquid supply source (not shown) via its connection to inlet opening 40. The DPT 10 also includes a poppet 50, which is capable of flushing the DPT 10 of fluid. The DPT 10 is capable of limiting the flow rate of a liquid, including sterilized liquid saline solution or the like, from the external sterilized-liquid supply source (not shown).

Not shown in FIG. 1 are the associated components of the pressure monitoring system that connect to the DPT 10. Typically, a signal receiving device such as a patient or cardiac output monitor includes cables and connectors that mate with the connector 32 and receive electrical signals indicative of fluid pressure detected by the DPT 10. Various monitors are available for this purpose and will not be further described herein, except below in the context of an interface feature of the present invention that permits identification by the monitor of the characteristics of the DPT 10.

Additionally, an in-dwelling catheter that provides the particular fluid to be measured attaches to one of the ports of the stopcock assembly 34, typically the port in line with the DPT 10 that is fitted with a luer connector. Many catheters may be used for pressure monitoring, and the specifics are well known in the art. Furthermore, the term "catheter" as used herein refers to any elongated structure for accessing a body cavity such as a blood vessel and provides a conduit through which fluid may pass. In the preferred embodiment, a saline solution provides a fluid "column" through which pressure pulses from the catheter lumen are transmitted, and a pressure transducer positioned along the fluid column monitors those pressure pulses. Devices for providing such access include cannulas, needles, sheaths, introducers, and other such structures, typically tubular.

Now with reference to FIG. 2, a housing 20 of the DPT 10 contains several components that are bonded or otherwise coupled together. The housing 20 includes a fluid chamber 60 and a pressure sensor 64, which extends from the housing 20 into the fluid chamber 60. The DPT 10 includes a cap 90 that secures the poppet 50 to the housing 20. The cap 90 can be made from a polycarbonate material that is ultrasonically welded to the housing 20.

The DPT 10 includes a poppet 50 and a capillary tube 70. The capillary tube 70 is bonded to an internal wall 22 of housing 20 with UV adhesive. The capillary 70 has a controlled flow rate for which the fluid A travels therethrough from the inlet channel 42 to the outlet channel 60. The DPT 10 comprises a by-pass channel 80 between the inlet channel 42 and outlet channel 60.

The poppet 50 can seal or close off the fluid chamber 60 from the by-pass channel 80. The fluid A entering inlet channel 42 from an external source (not shown) must pass through the capillary tube 70 to the fluid chamber 60. When the by-pass channel 80 is sealed, the fluid A travels through the capillary tube 70 at a continuous and slow rate in order to prevent the fluid from coagulating in the blood circuit. The capillary tube 70 restricts the flow rate of the fluid A. The size and shape of the capillary tube correspond to a desired flow rate.

When the poppet 50 is pulled away from the housing 20 in a direction B, the poppet 50 allows the by-pass channel 80 to be in fluid communication with the inlet channel 42 and the outlet channel 60. The fluid A from the external supply source flows through the by-pass channel 80 and into the outlet channel 60. The flow through the by-pass channel allows for fast-flow flushing of the DPT 10.

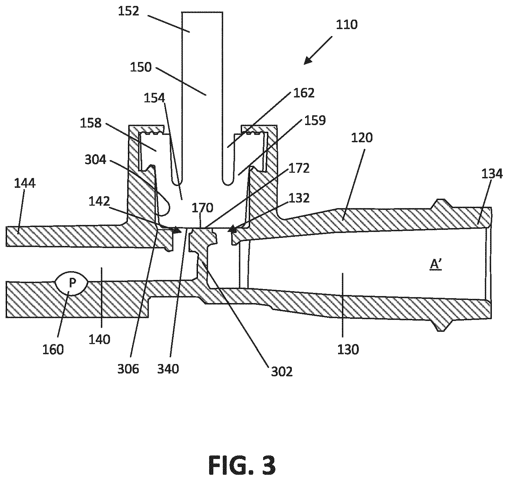

FIGS. 3-6 illustrate an exemplary embodiment of a disposable pressure transducer (DPT) 110. The DPT 110 can take a wide variety of different forms. In the example illustrated by FIG. 3, the DPT 110 includes a housing 120, a poppet 150, and a pressure sensor 160.

In one exemplary embodiment, the DPT 110 does not include a capillary tube 70 (See Prior Art Figure 2). Instead, a flow restrictor 170 is integrally formed by one or more of a poppet 150 and a portion of a housing 120. Such a flow restrictor 170 can take a wide variety of different forms. For example, the flow restrictor can be formed at an interface of the poppet 150 and a valve seat 172 of the housing 120, such as a flow channel in a surface of the valve seat 300, a flow channel in a surface of the poppet 150, or a flow channel or passage defined by both the valve seat 172 and the poppet 150. The integral flow restrictor 170 can also be a passage or passages through the housing 120, such as a passage or passages through a portion 302 of the housing 120 below the valve seat 172, or a passage or passage through the poppet 150, such as a passage that extends from the inlet port 132 to the outlet port 142. Any structure that is integral with the poppet 150 and/or housing 120 that replaces the capillary function of the prior separate capillary tube can be used.

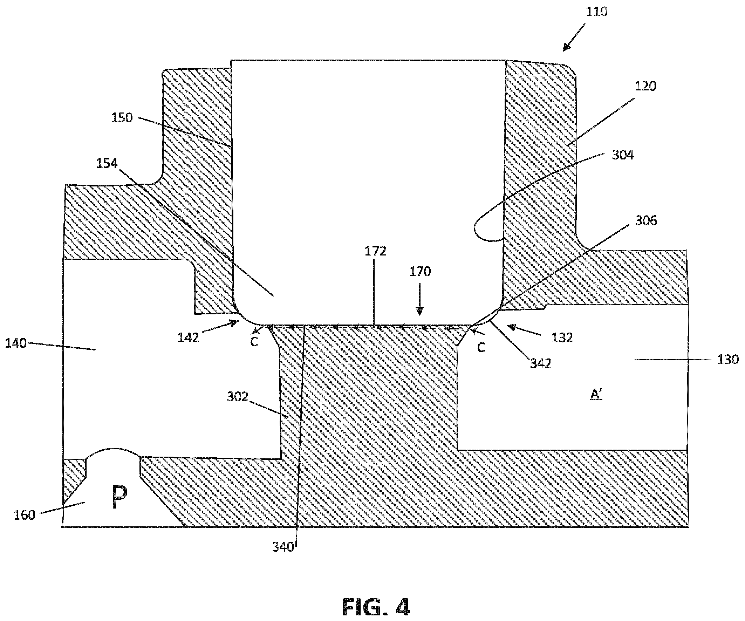

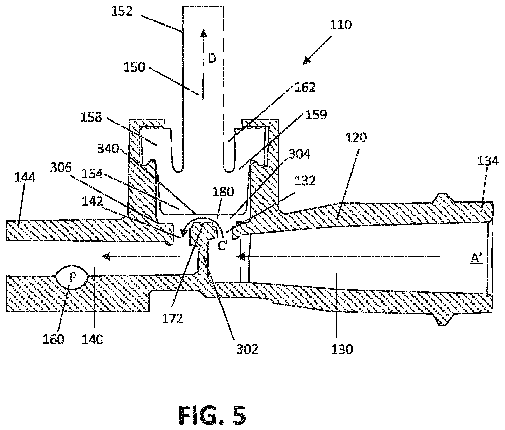

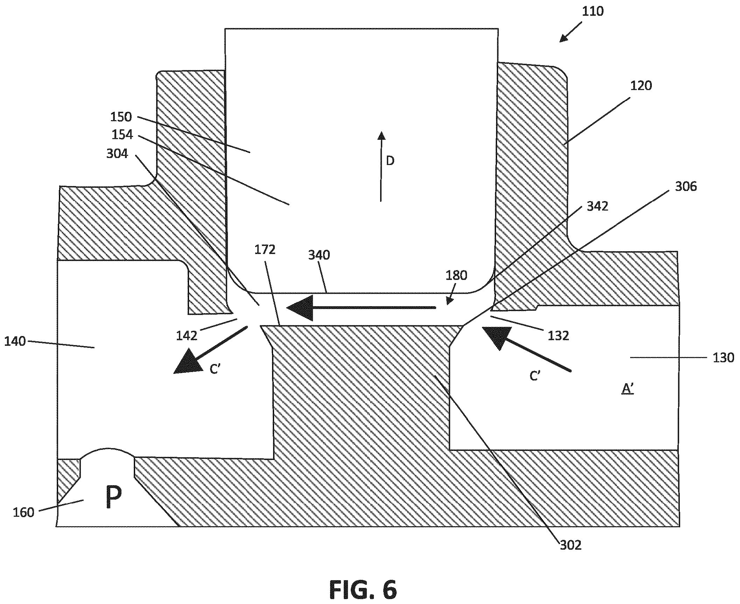

The housing 120 can take a wide variety of different forms. In one exemplary embodiment, the housing 120 includes an inlet passage 130, an outlet passage 140, a valve seat 172, and a poppet cavity 304. Referring to FIG. 4, when the poppet 150 is closed against the valve seat 172, fluid can flow slowly from the inlet passage 130, through the flow restrictor 170, and out the outlet passage 140. Referring to FIGS. 5 and 6, when the poppet 150 is open, fluid can flow rapidly from the inlet passage 130, through the poppet cavity 304 (in the space between the poppet 150 and the valve seat 172), and out the outlet passage 140.

The inlet passage 130, outlet passage 140, and valve seat 172 can take a wide variety of different forms. In one exemplary embodiment, the valve seat 172 includes an inlet port 132 and/or an outlet port 142. In one exemplary embodiment, the inlet port 132 is in fluid communication with the inlet passage 130, the poppet cavity 304, and the flow restrictor 170. In one exemplary embodiment, the outlet port 142 is in fluid communication with the outlet passage 140, the poppet cavity 304, and the flow restrictor 170.

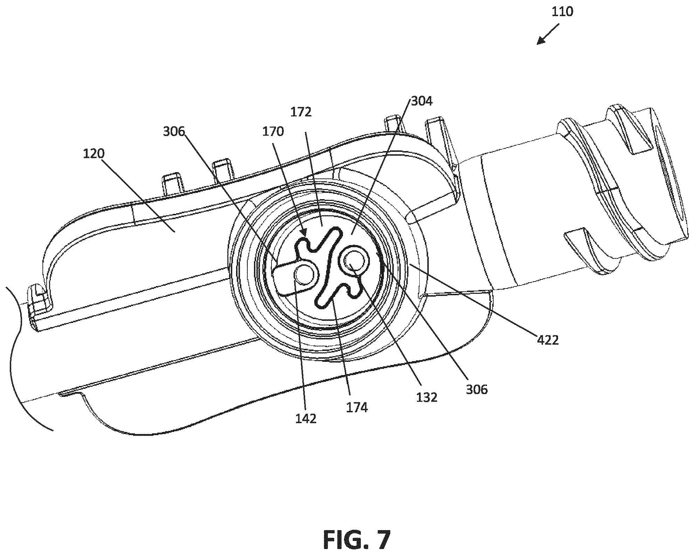

The inlet port 132 and the outlet port 142 can take a wide variety of different forms. For example, the inlet port 132 and/or the outlet port 142 can be perpendicular or generally perpendicular to surface of the valve seat 172 as illustrated by the example of FIG. 3. The inlet port 132 and/or the outlet port 142 can extend at an angle into the poppet cavity 304 as illustrated by the example of FIG. 4. The inlet port 132 and/or the outlet port 142 can be inward of an outer periphery 306 of the valve seat 172, like the inlet port 132 illustrated by FIGS. 3 and 7. The inlet port 132 and/or the outlet port 142 can extend to the outer periphery 306, like the inlet and outlet ports 132,142 illustrated by FIG. 4 and the outlet port 142 illustrated by FIGS. 3 and 7. One or both of the inlet port 132 and the outlet port 142 can be configured to be sealed off by the poppet 150, when the poppet 150 is closed, like the inlet port 132 illustrated by FIG. 3 and the inlet and outlet ports illustrated by FIG. 4. One of the inlet port 132 and the outlet port 142 can be configured to be unsealed (i.e. not blocked off) by the poppet 150, when the poppet 150 is closed, like the outlet port 142 illustrated by FIG. 3.

The poppet 150 can take a wide variety of different forms. In one exemplary embodiment, the poppet 150 includes a sealing portion 154, an actuator or control portion 152, a mounting portion 158, and a flexing portion 159. Referring to FIG. 3, the poppet 150 is connected to the housing 120 by securing the mounting portion 158 to the housing 120. The sealing portion 154 is connected to the mounting portion 158 by the flexing portion 159. The sealing portion 154 is also connected to the actuator or control portion 152. Referring to FIG. 3, in one exemplary embodiment the flexing portion 159 biases the sealing portion 154 against the valve seat 172. Referring to FIG. 5, the poppet 150 is opened by pulling on the actuator or control portion 152. This pulls the sealing portion 154 away from the valve seat 172 and flexes the flexing portion 159. When the actuator or control portion 152 is released, the flexing portion 159 returns the sealing portion 154 to engagement with the valve seat 172.

The DPT 110 includes a pressure sensor 160 to measure the fluid pressure in the outlet channel 140. The pressure sensor can take a wide variety of different forms. In one exemplary embodiment, the pressure sensor is a silicon pressure sensor that can have a thin monocrystalline silicon diaphragm. The pressure sensor can have four terminals. Acceptable silicon pressure sensors are commercially available from Motorola, Inc. More details on acceptable pressure transducers are disclosed in U.S. Pat. Nos. 4,539,998, and RE 33,518. The pressure sensor can include a temperature compensation circuit for compensating the sensed pressure in the fluid based upon the temperature of the fluid.

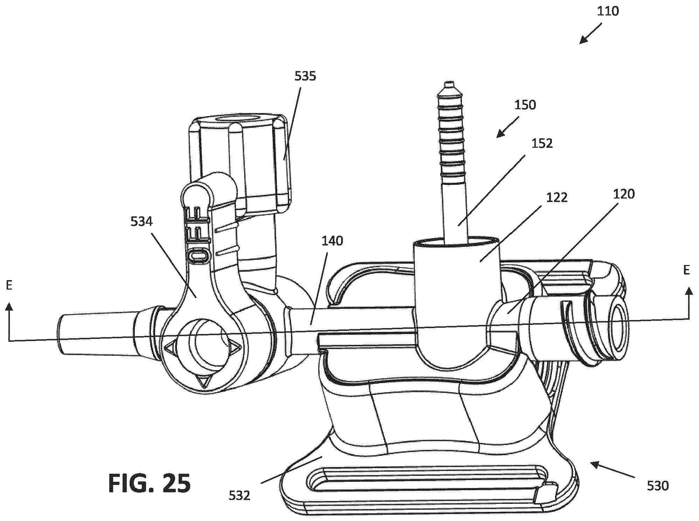

Referring to FIGS. 3-5, the inlet channel 130 in fluid communication with the inlet port 132 and an external liquid source (not shown), such as an intra venous bag filled with fluid. The inlet channel 130 extends from an inlet opening 134 to the inlet port 132. The outlet channel 140 is in fluid communication with the outlet port 142. The outlet channel 140 can extend from the outlet port 142 to an optional stopcock assembly 534 (See FIG. 25).

With reference to FIGS. 3-4, the DPT 110 is in the "engaged or closed state" where the sealing portion 154 of the poppet 150 is coupled with or sealed against valve seat 172, to seal off the inlet port 132 and/or the outlet port 142. In the configuration illustrated by FIG.3, in the "engaged state" a face 340 of the sealing portion 154 seals against the valve seat 172, around the inlet port 132, to seal off the inlet port 132. In the configuration illustrated by Figure 4, in the "engaged state" an annular surface 342 of the sealing portion 153 seals against the valve seat 172, around the inlet port 132 and around the outlet port 142. Referring to Figure 4, the fluid A' travels through the first flow path C, from the inlet channel 130 to the outlet channel 140, via the flow restrictor 170, when the poppet 150 is engaged with the valve seat 172 of the housing 120.

In various embodiments, in the "engaged state" where the poppet 150 seals with the valve seat 172 of the housing 120, fluid A' from an external source (not shown) is in communication with a first flow path C and to the outlet channel 140. The fluid A' in first flow path C can flow through various structures, including the inlet channel 130, inlet port 132, flow restrictor 170, outlet port 142, and outlet channel 140. As long as the pressure in the inlet 130 is higher than the pressure in the outlet 140 to a sufficient degree to flow through the restrictor, the fluid A' in first flow path C flows from the external source through the inlet channel 130 and to the inlet port 132. The fluid A' in first flow path C then travels from the inlet port 132 through the flow restrictor 170. The fluid A' in first flow path C then travels through the outlet port 142 and through the outlet channel 140.

Still referring to FIGS. 3 and 4, the flow of the fluid A' in first flow path C is restricted due to the sealing portion 154 of the poppet 150 being coupled with the valve seat 172. This coupling closes the by-pass space 180 (FIG. 5) of the poppet cavity 304. As a result, the flow restrictor 170 is the only way for the fluid A' to travel from the inlet port 132 to the outlet port 142. In various embodiments, the flow restrictor 170 is formed from the same portion of the housing 120 that forms the valve seat 172. The flow restrictor 170 can slow or otherwise control the rate at which the fluid A' enters the outlet port 142. The slow, restricted flow prevents fluid from coagulating in the DPT and an intravenous line that is connected to the DPT.

A wide variety of flow rates through the restrictor 170 can be selected. In various embodiments, the fluid A' traveling through the first flow path C can have a flow rate between about 1 cc/hr to about 10 cc/hr. In various embodiments, the fluid A' traveling through the first flow path C can have a flow rate between about 1.5 cc/hr to about 8 cc/hr. In various embodiments, the fluid A' traveling through the first flow path C can have a flow rate between about 2 cc/hr to about 6 cc/hr. In various embodiments, the fluid A' traveling through the first flow path C can have a flow rate between about 2.5 cc/hr to about 3.5 cc/hr. In various embodiments, the fluid A' traveling through the first flow path C can have a flow rate of about 3 cc/hr or 3 cc/hr.

With reference to FIGS. 5 and 6, the DPT 110 is in the "disengaged state" and is configured to allow fluid A' to flow in a second flow path C' from the inlet channel 130 through the by-pass passage 180 (between the sealing portion 154 and the valve seat 172) of the poppet cavity 304, to the outlet channel 140. To place the DPT in the "disengaged state," the actuator or shaft 152 is pulled in the direction D. The actuator or shaft 152 pulls the sealing portion 154 away from the valve seat and flexes the flexible portion 159. The resulting space between the sealing portion 154 of the poppet 150 and the valve seat 172 comprises the by-pass channel 180 through which fluid A' can flow through and flush the DPT 110.

The second flow path C' can include the inlet channel 130, inlet port 132, by-pass channel 180, outlet port 142, and outlet channel 140. In various embodiments, in the disengaged state, the flow path B' can include the flow restrictor 170, since a portion of the fluid A' can still flow through the flow restrictor 170.

As mentioned above, the by-pass channel 180 comprises a space defined by the poppet 150 and the valve seat 172 of the housing 120. The fluid A' flowing through flow path C' can result in fast-flow flushing and over-pressure relief of the DPT 110. The by-pass channel 180 can be various sizes based on the how far the popped 150 is pulled in direction D from the valve seat 172. For example, if the poppet 150 is pulled from the valve seat 172 with less force, then the volume of the by-pass channel 180 will not be as large and the resulting amount fluid A' traveling through the by-pass channel 180 will be less. Conversely, if the poppet 150 is pulled from the housing 120 with a greater force, then the by-pass channel 180 will be a greater volume and the resulting amount of fluid A' traveling through the by-pass channel 180 will increase. The amount of fluid A' traveling through the by-pass channel 180 to the outlet channel 140, and the flow rate thereof, can therefore be proportional to the size of the by-pass channel 180.

In various embodiments, the fluid A' traveling through the flow path C' can have a flow rate between about 5 cc/min to about 250 cc/min. In various embodiments, the fluid A' traveling through the first flow path C' can have a flow rate between about 20 cc/ min to about 225 cc/min. In various embodiments, the fluid A' traveling through the first flow path C' can have a flow rate between about 50 cc/ min to about 200 cc/min. In various embodiments, the fluid A' traveling through the first flow path C' can have a flow rate between about 70 cc/ min to about 175 cc/min. In various embodiments, the fluid A' traveling through the first flow path C' can have a flow rate between about 80 cc/ min to about 150 cc/min. In various embodiments, the fluid A' traveling through the first flow path C' can have a flow rate between about 100 cc/ min to about 115 cc/min. In various embodiments, the fluid A' traveling through the first flow path C' can have a flow rate of about 110 cc/min.

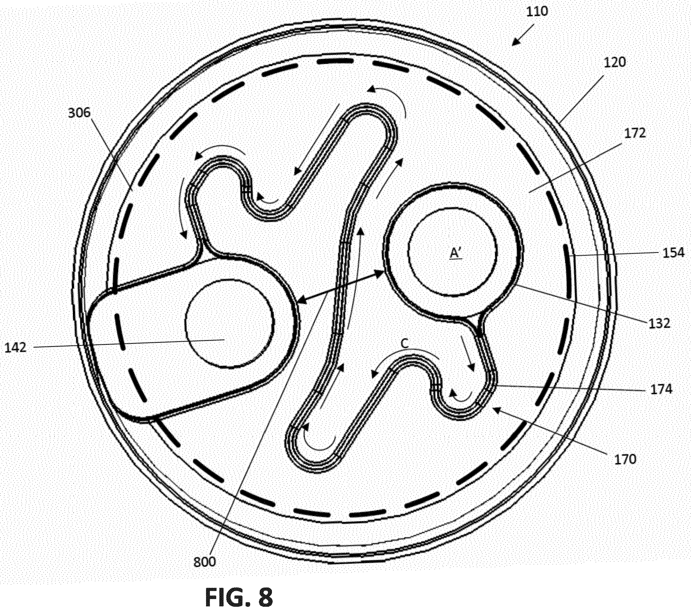

As mentioned above, the flow restrictor 170 can take a wide variety of different forms. Referring to FIGS. 7 and 8, in the presently claimed invention, the flow restrictor 170 comprises a flow restrictor channel 174. In the presently claimed invention, the flow restrictor channel 174 extends into the valve seat 172, as illustrated. In other examples, the flow restrictor channel 174 can extend into the face 340 of the poppet 150. The flow restrictor channel 174 can be a variety of shapes and have a variety of lengths, widths, and depths to best optimize the flow rate of the fluid A' flowing through. For example, in cross section, the flow restrictor channel 174 can comprise a rounded, rectangular, or trapezoidal shape. The flow restrictor 170 can include a predetermined shape, length, width, or depth, or combination thereof, based on the flow rate of the fluid A' desired.

With reference to FIG. 7, the flow restrictor 170 can have numerous turns to increase the length of the flow restrictor channel 174 between the inlet port 132 and the outlet port 142. Increasing the length of the flow restrictor channel 174 decreases the flow rate through the flow restrictor. As such, for a set or desired flow rate, a size or cross-sectional area of the flow restrictor channel 174 can be increased if the length of the flow restrictor channel 174 is also increased. In one exemplary embodiment, the length of the flow restrictor channel is between 2 and 20 times the distance 800 between the inlet port 132 and the outlet port 142, such as between 3 and 10 times the distance 800 between the inlet port 132 and the outlet port 142, such as between 4 and 6 times the distance 800 between the inlet port 132 and the outlet port 142.

With reference to FIG. 8, a top view of the valve seat 172 of the DPT 110 in the engaged state is illustrated, where the sealing portion 154 of the poppet 150 (illustrated as a dotted line) presses against the valve seat 172 of the housing 120. In the engaged state, the fluid A' traveling from the inlet port 132 continuously flows through the first flow path C through the flow restrictor channel 174 to the outlet port 142.

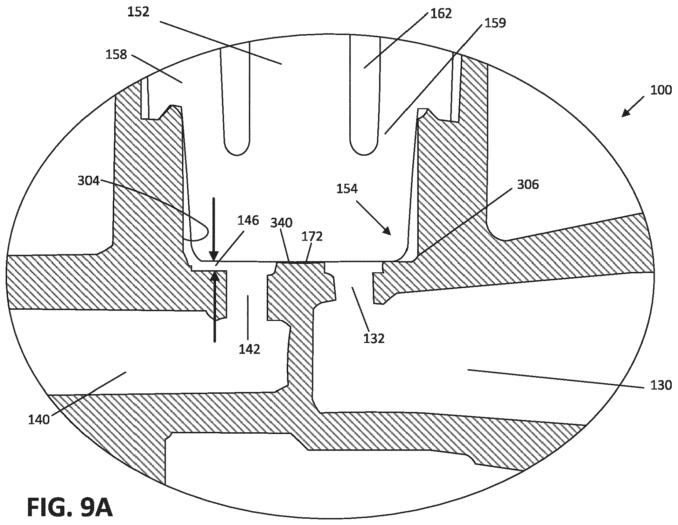

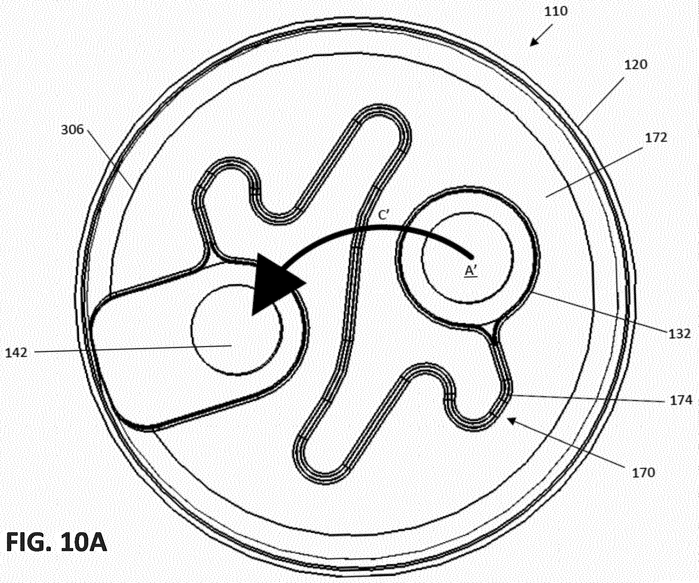

In various embodiments, and with reference to FIG. 9A, in the engaged state, end surface 340 of the sealing portion 154 presses against the valve seat 172 and seals off the inlet port 132. However, the end surface 340 of the sealing portion 154 does not seal off the outlet port 142. As a result, when a predetermined pressure (referred to as "overpressure") is provided in the inlet channel 130, the "overpressure" acts on the end surface 340 and forces the sealing portion 154 upward. This opens the poppet 150 to allow fluid A' to allow for flow through the bypass channel 180 (See FIG. 5-6) to the outlet port 142 (See Figure 10A) and thereby reduce the pressure in the inlet channel. As such, the poppet 150 can be opened both by pulling on the actuator or shaft 152 and by application of an overpressure to the inlet channel 130.

With reference to FIG. 10A, a top view of the DPT 110 configuration of FIG. 9A in the disengaged state is illustrated. The sealing portion 154 of the poppet 150 moved away from the valve seat 172 of the housing 120. As shown in FIG. 6, the volume of the by-pass channel 180 is determined by the distance between the sealing portion 154 of the poppet 150 and the valve seat 172. In the disengaged state, the fluid A' from the inlet port 132 flows through the by-pass channel 180 via the second flow path C' to the outlet port 142 at a greater rate than that of in the engaged state.

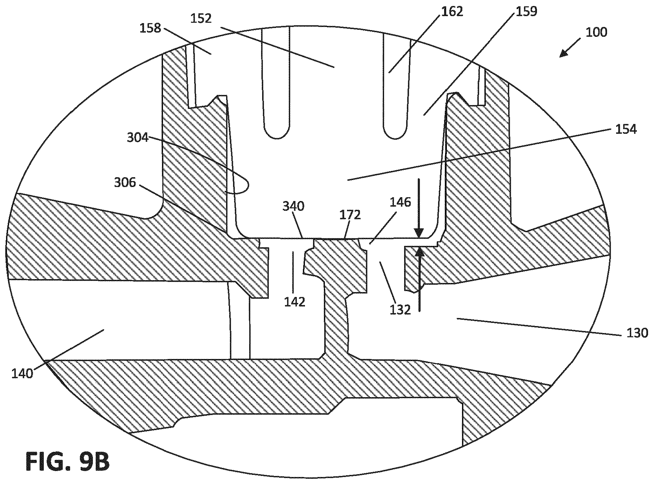

In various embodiments, and with reference to FIG. 9B, in the engaged state, end surface 340 of the sealing portion 154 presses against the valve seat 172 and seals off the outlet port 132. However, the end surface 340 of the sealing portion 154 does not seal off the outlet port 132. As a result, when a predetermined pressure (referred to as "overpressure") is provided in the outlet channel 140, the "overpressure" acts on the end surface 340 and forces the sealing portion 154 upward. This opens the poppet 150 to allow fluid A' to allow for backflow (flow in the direction opposite to the arrows of FIGS. 5, 6 and 10B) through the bypass channel 180 (See FIG. 5-6) to the inlet port 132 and thereby reduce the pressure in the outlet channel 140. As such, the poppet 150 can be opened both by pulling on the actuator or shaft 152 and by application of an overpressure to the outlet channel 140.

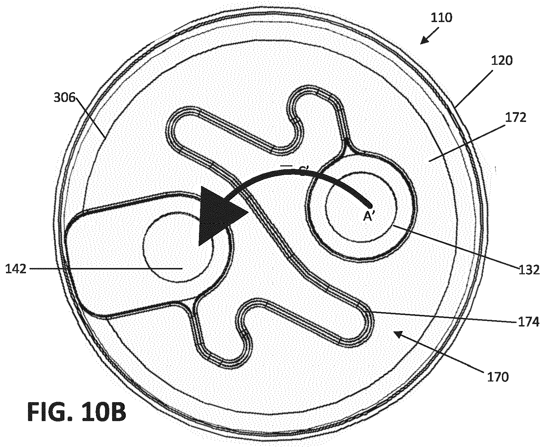

With reference to FIG. 10B, a top view of the DPT 110 of the FIG. 9B configuration in the disengaged state is illustrated. The sealing portion 154 of the poppet 150 moved away from the valve seat 172 of the housing 120. As shown in FIG. 6, the volume of the by-pass channel 180 is determined by the distance between the sealing portion 154 of the poppet 150 and the valve seat 172. In the disengaged state, the fluid A' from the inlet port 132 flows through the by-pass channel 180 via the second flow path C' to the outlet port 142 at a greater rate than that of in the engaged state.

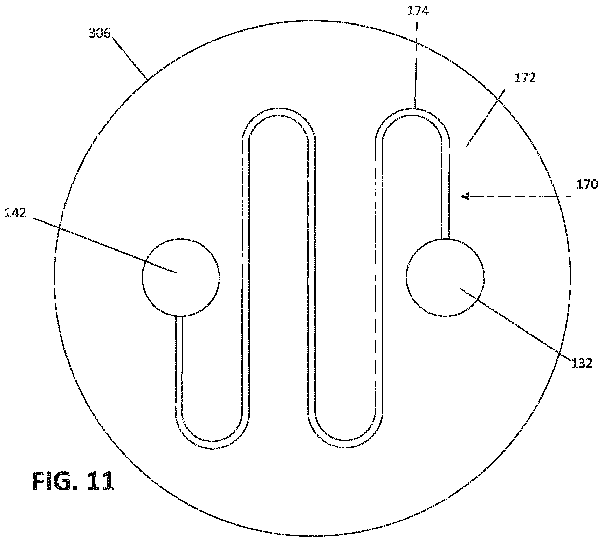

The path of the flow restrictor channel 174 can take a wide variety of different forms. FIG. 11 illustrates another path of a flow restrictor channel 174. The flow restrictor channel 174 of the flow restrictor 170 can have a snake-like shape with any number of turns. The length, width, and depth of the flow restrictor channel 174 can be predetermined to coincide with a specific flow rate, for a specific pressure differential between the inlet port 132 and the outlet port 142. Holding the width and depth constant, increasing the length of the flow restrictor channel 174 results in a slower flow rate of the fluid A' through the flow restrictor 170. Conversely, the length of the flow restrictor channel 174 can be decreased if a faster flow rate is preferred. In various embodiments, the flow restrictor channel 174 can have a direct path between the inlet port 132 and the outlet port 142. However, the cross-sectional are of the flow restrictor channel 174 will decrease to accommodate the shorter path.

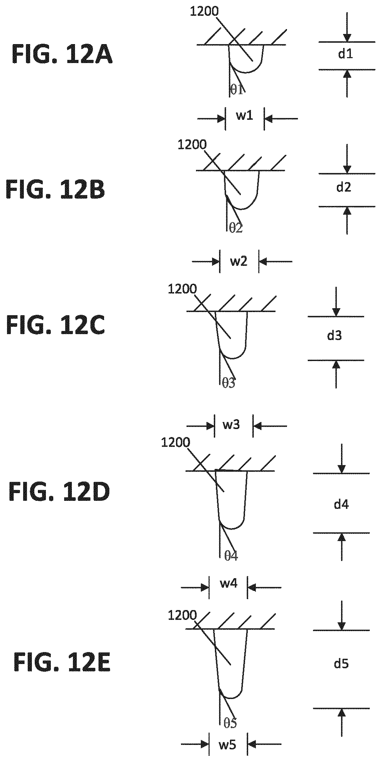

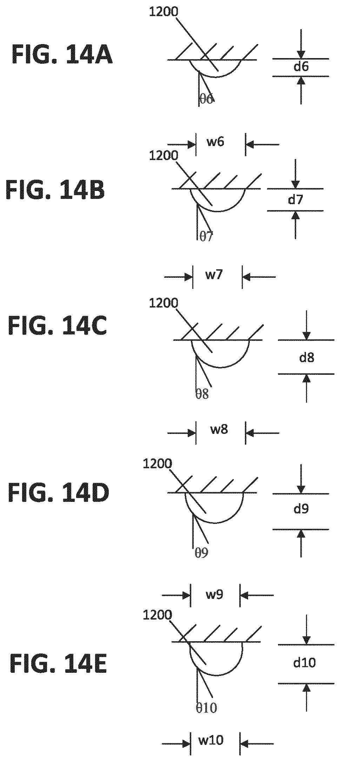

With reference to FIGS. 12A-12E and 14A-14E, various profiles of projections 1200 used to mold the flow restrictor channel 174 of the flow restrictor 170 are shown. The flow restrictor channel 174 of the flow restrictor 170 can be molded using projections of various sizes and shapes, resulting in shapes of the flow restrictor corresponding to that of the pins. It should be apparent that the top 1300 of the molded flow restrictor channel 174 can be larger than the base of the flow restrictor projection. In FIGS. 12A-12E, the depth of the flow restrictor channel 174 (i.e. the height of the projection 1200) is greater than the width of the flow restrictor channel 174 (based on the width of the projection 1200). In FIGS. 14A-14E, the depth of the flow restrictor channel 174 (i.e. the height of the projection 1200) is less than the width of the flow restrictor channel 174 (based on the width of the projection 1200).

In various embodiments, the depth of the flow restrictor channel 174 can be between 0.0127mm (0.0005 inches) and 0.2032mm (0.0080 inches). In various embodiments, the depth of the flow restrictor channel 174 can be between 0.0254mm (0.0010 inches) and 0.1778mm (0.0070 inches). In various embodiments, the depth of the flow restrictor channel 174 can be between 0.0508mm (0.0020 inches) and 0.127mm (0.0050 inches). In various embodiments, the depth of the flow restrictor channel 174 can be between 0.0762mm (0.0030 inches) and 0.1016mm (0.0040 inches). In various embodiments, the depth of the flow restrictor channel 174 can be 0.0889mm (0.00350 inches).

In various embodiments, the width of the flow restrictor channel 174 can be between 0.0127mm (0.0005 inches) and 0.2032mm (0.0080 inches). In various embodiments, the width of the flow restrictor channel 174 can be between 0.0254mm (0.0010 inches) and 0.1778mm (0.0070 inches). In various embodiments, the width of the flow restrictor channel 174 can be between 0.0508mm (0.0020 inches) and 0.127mm (0.0050 inches). In various embodiments, the width of the flow restrictor channel 174 can be between 0.0762mm (0.0030 inches) and 0.1016mm (0.0040 inches). In various embodiments, the width of the flow restrictor channel 174 can be 0.0889mm (0.00350 inches).

In various embodiments, the width of the flow restrictor channel 174 is greater than its depth. With reference to FIG. 12A, the projection 1200 used to make the flow restrictor channel 174 has a depth (d1) of 0.04572mm (0.0018 inches), a width (w1) of 0.0508mm (0.0020 inches), and an angle (θ1) of 5°. With reference to FIG. 12B, the projection 1200 used to make the flow restrictor channel 174 has a depth (d2) of 0.06096mm (0.00240 inches), a width (w2) of 0.0508mm (0.0020 inches), and an angle (θ2) of 5°. With reference to FIG. 12C, the projection 1200 used to make the flow restrictor channel 174 has a depth (d3) of 0.0762mm (0.0030 inches), a width (w3) of 0.0508mm (0.0020 inches), and an angle (θ3) of 5°. With reference to FIG. 12D, the projection 1200 used to make the flow restrictor channel 174 has a depth (d4) of 0.09398 (0.00370 inches), a width (w4) of 0.0508mm (0.0020 inches), and an angle (θ4) of 5°. With reference to FIG. 12E, the projection 1200 used to make the flow restrictor channel 174 has a depth (d5) of 0.1143mm (0.00450 inches), a width (w5) of 0.0508mm (0.0020 inches), and an angle (θ5) of 5°.

With reference to FIG. 3, the poppet 150 exerts a force on the valve seat 172 of the housing 120. In various embodiments, the force that the poppet 150 exerts on the valve seat 172 of the housing 120 (i.e. the "pre-load") can have an effect on the flow rate of fluid A' through the flow restrictor 170. For example, the sealing portion 154 can be made from a soft and/or flexible material that can comprise one or more of rubber, a synthetic rubber, a synthetic rubber-like material, silicone, Teflon, etc. This soft material can push into the flow restrictor channel 174. As a result, the cross-sectional area of the flow restrictor channel 174 is reduced, reducing the flow rate through the channel 174.

As the pre-load of the poppet 150 on the valve seat 170 increases, the flow rate of fluid A' through the flow restrictor 170 decreases. For example, with a smaller pre-load, the sealing portion 154 of the poppet 150 may rest on the valve seat 172 such that the sealing portion 154 does not enter any portion of the flow restrictor channel 174. However, as the pre-load increases, the sealing portion 154 of the poppet 150, which may be deformable, may be pushed into a portion of the flow restrictor channel 174 and decrease the volume of the flow restrictor channel 174 that the fluid A' can travel through. This can result in a slower flow rate of fluid A' through the flow restrictor 170. The deformation of the poppet material into the channel can be affected by a variety of factors, including the width of the flow restrictor channel, the composition of the poppet 150, the composition of the housing 120, the force at which the poppet is pressed against the valve seat.

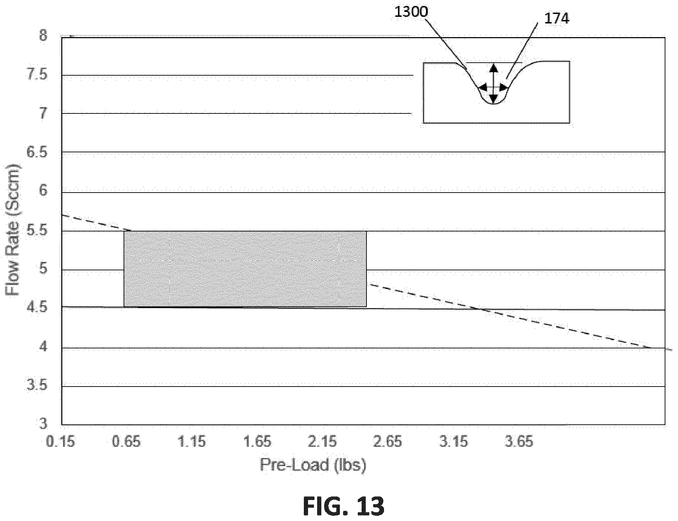

With reference to FIG. 13, the relationship between the flow rate (Sccm) and the pre-load of the poppet (lbs) is shown for the channel 174 made from the projection depicted in FIG. 12C. As mentioned above, in Figure C the projection 1200 used to make the flow restrictor channel 174 has a depth (d3) of 0.0762mm (0.0030 inches), a width (w3) of 0.0508mm (0.0020 inches), and an angle (θ3) of 5°. In one exemplary embodiment, the projection illustrated by Figure 12C makes the channel 174 illustrated at the top of FIG. 13, which has a depth that is greater than the width. At a pre-load of 0.65 lbs, the flow rate is about 5.5 Sccm. At a preload of 2.65 lbs, the flow rate of is about 4.75 Sccm.

In various embodiments, the width of the flow restrictor channel 174 is greater than its depth. With reference to FIG. 14A, the projection 1200 used to make the flow restrictor channel 174 has a depth (d6) of 0.02794 (0.00110 inches), a width (w6) of 0.1016mm (0.004 inches), and an angle (θ6) of 5°. With reference to FIG. 14B, the projection 1200 used to make the flow restrictor channel 174b' has a depth (d7) of 0.0556 (0.00140 inches), a width (w7) of 0.1016mm (0.004 inches), and an angle (θ7) of 5°. With reference to FIG. 14C, the projection 1200 used to make the flow restrictor channel 174h has a depth (d8) of 0.04318 (0.00170 inches), a width (w8) of 0.1016mm (0.004 inches), and an angle (θ8) of 5°. With reference to FIG. 14D, the projection 1200 used to make the flow restrictor channel 174i has a depth (d9) of 0.04826 (0.00190 inches), a width (w9) of 0.1016mm (0.004 inches), and an angle (θ9) of 5°. With reference to FIG. 14E, the projection 1200 used to make the flow restrictor channel 174j has a depth (d10) of 0.05588 (0.00220 inches), a width (w10) of 0.1016mm (0.004 inches), and an angle (θ10) of 5°.

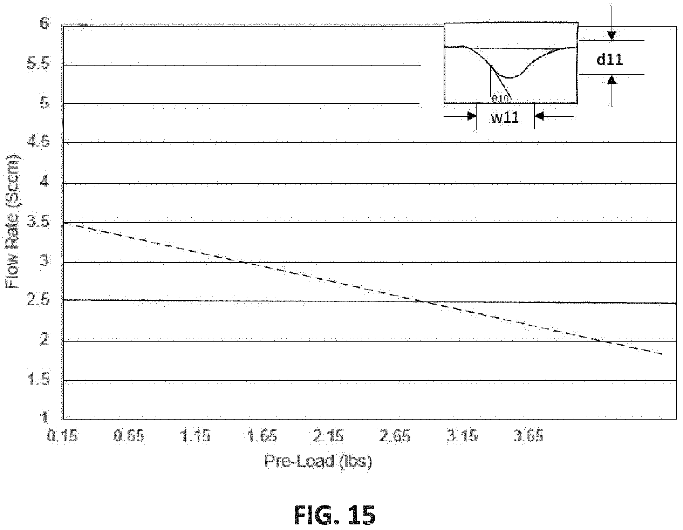

With reference to FIG. 15, the relationship between the flow rate (Sccm) and the pre-load of the poppet (lbs) is shown for a flow restrictor channel 174 having a depth (d11) of 0.0508mm (0.002 inches), a width (w11) of 0.0762mm (0.003 inches), and an angle (θ11) of 5°. As such, the flow restrictor has a width that is greater than the depth. At a pre-load of about 0.65 lbs, the flow rate through the flow restrictor channel is about 3.4 Sccm. At a preload of about 2.5 lbs, the flow rate is about 2.5 Sccm.

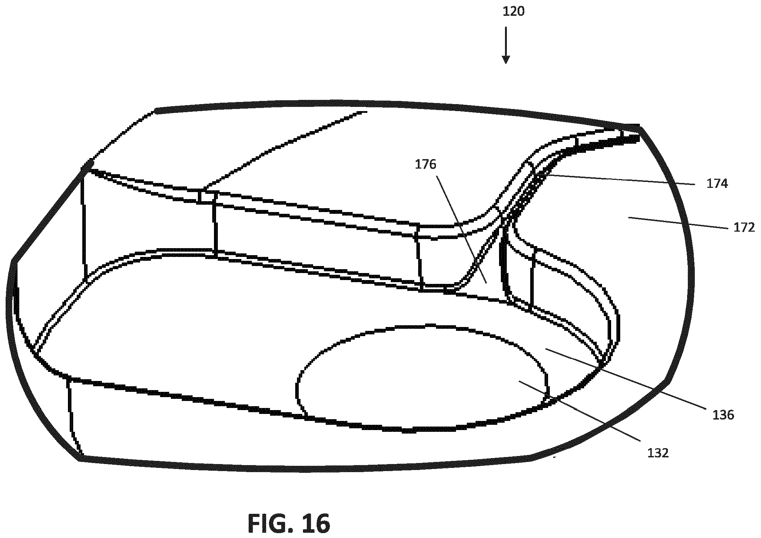

With reference to FIG. 16, the body 120 of the presently claimed DPT 110 includes a ramp 176 between the flow restrictor channel 174 and the inlet port 132 and/or outlet port 142. In various embodiments, the ramp 176 connects the flow restrictor channel 174 to a recess 136 that is connected to the inlet 132. In various embodiments, the ramp 176 can connect the flow restrictor channel 174 to an outlet recess that is connected to an outlet port 142. The optional inlet recess 136 and/or an outlet recess can be built into the housing 122 and surround the inlet port 132 and outlet port 142, respectively. The ramp 176 can increase the space for the fluid A' to travel into the flow restrictor channel 172. The extra space provided by ramp 176 can reduce the risk of a blockage of the entrance or exit of the flow restrictor channel 174 caused by load that presses the sealing portion 154 of the poppet into the inlet port 132 and/or the outlet port 142.

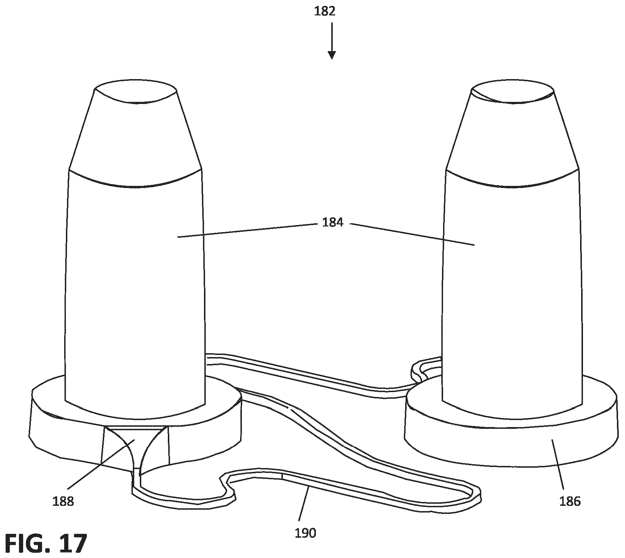

With reference to FIG. 17, a mold having an insert 182 can be used to form various portions of the DPT 110. For example, with reference to FIG. 16 and 17, the mold insert 182 can be used to create one or more of the inlet port 132, inlet recess 136, an inlet ramp 176, the flow restrictor channel 172. The mold insert 182 can also be used to create an outlet recess, an outlet port 142, an outlet ramp, and an outlet port 142. The mold insert 182 can include a pin 184 that corresponds to, and is the negative of, at least one of inlet port 132 and the outlet port 142 of the DPT 110. The mold 182 can include a shoulder or ring 186 that corresponds to, and is the negative of, at least one of inlet recess 136 and an outlet recess of the DPT 110. The mold insert 182 can include a ramp portion 188 that corresponds to, and is the negative of, the ramp 176 of the DPT 110. The mold 182 can include an elevated tortuous projection 190 to correspond to, and is the negative of, the flow restrictor channel 172 of the DPT 110.

The poppet 150 can take a wide variety of different forms. The sealing portion 154 can take a wide variety of different forms and can be made from a wide variety of different materials. The sealing portion 154 can be configured such that an end face 340 of the seal portion provides the seal (See FIG. 3) or such that an outer circumferential portion 342 provides the seal. The seal portion can be made of a single material or a portion of the sealing portion 154 that makes contact with the valve seat 170 can be made from a first, sealing material, and other portions of the sealing portion 154 can be made from another material. one or more of rubber, a synthetic rubber, a synthetic rubber-like material, silicone, Teflon, etc.

The flexing portion 159 can take a wide variety of different forms. The flexing portion 159 can be integrally formed with the sealing portion 154 as illustrated, or the flexing portion 159 can be a separate component that presses the sealing portion 154 against the valve seat 170. In one exemplary embodiment, a void 162 creates flexing portion 159. The flexing portion 159 can be made from a variety of different materials. For example, the flexing portion 159 can be made from one or more of rubber, a synthetic rubber, a synthetic rubber-like material, silicone, Teflon, etc.

The actuator 152 can take a wide variety of different forms. The actuator 152 can have the illustrated shaft configuration or can have any configuration that allows a user to move the sealing portion from the closed position to the open position. The actuator 152 can be integrally formed with the sealing portion 154 as illustrated, or the actuator 152 can be a separate component that is connected to the sealing portion. The flexing portion 159 can be made from a variety of different materials. For example, the flexing portion 159 can be made from one or more of metal, rigid plastic, rubber, a synthetic rubber, a synthetic rubber-like material, silicone, Teflon, etc.

The mounting portion 158 can take a wide variety of different forms. In the illustrated examples, the mounting portion 158 is both used to secure the poppet to the housing 120 and seal the poppet 150 in the poppet cavity. The mounting portion 158 can have the illustrated ring configuration or can have any configuration that facilitates securing the poppet to the housing 120 and sealing of the poppet 150 in the poppet cavity. The mounting portion 158 can be integrally formed with the flexing portion 159 as illustrated, or the mounting portion 158 can be a separate component that is connected to the sealing portion or that connects the flexing portion to the housing 120. The mounting portion 158 can be made from a variety of different materials. For example, the mounting portion 158 can be made from one or more of metal, rigid plastic, rubber, a synthetic rubber, a synthetic rubber-like material, silicone, Teflon, etc.

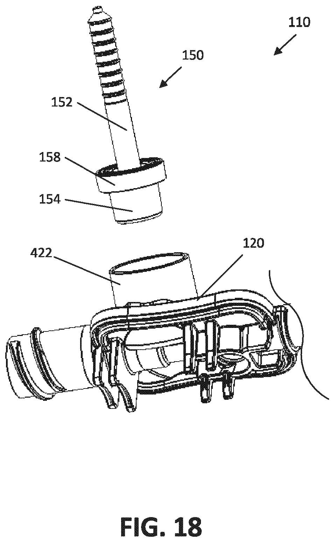

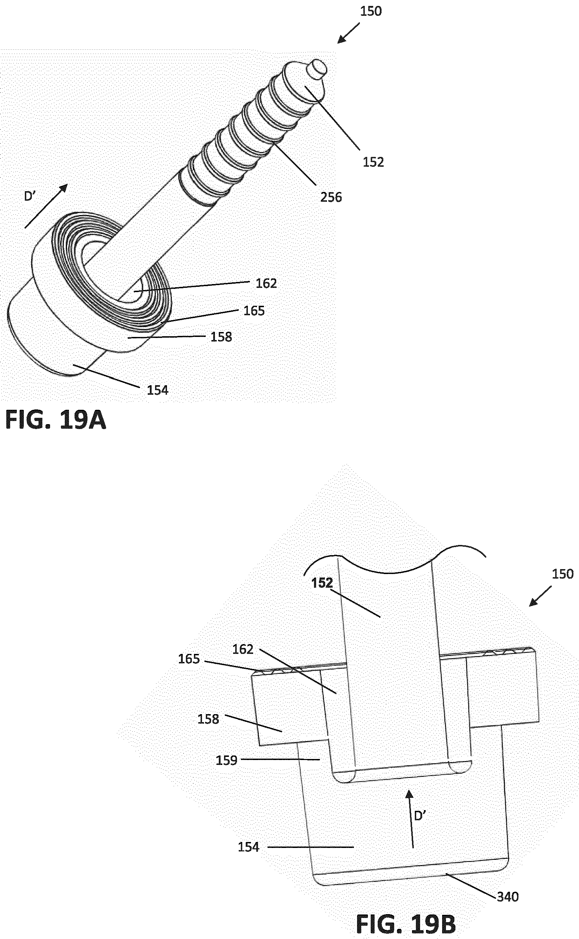

FIGS. 18, 19A and 19B illustrate an exemplary embodiment of a poppet 150. With reference to FIG. 18, the poppet 150 is shown separate from the housing 120 of the DPT 110. With reference to FIGS. 19A-B, the poppet 150 includes an actuator 152 and a sealing portion 154. Referring to FIG. 19A, the actuator 152 of the poppet 150 can include one or more ribs 256 that extend radially outward from the actuator 152. The ribs 256 can be positioned at or near the end of the actuator 152. The ribs 256 can aid in ensuring a secure grip used when opening or otherwise handling the poppet 250.

Referring to FIGS. 19A-B, the mounting portion 158 of the poppet 150 is a radially outwardly extending ring. The ring-shaped mounting portion 158 is used to secure the poppet 150 to the housing 120 of the DPT 110. In the example illustrated by FIGS. 19A and 19B, the ring-shaped mounting portion 158 includes a plurality of concentric ring protrusions 165. The concentric ring protrusions 165 extend axially from an end of the mounting portion 158. The concentric ring protrusions 165 can seal with the housing 120.

Still referring to FIGS 19A and 19B, the poppet 150 can include a void or cutout 162 between the ring-shaped mounting portion 158 and the actuator shaft 152. The void or cutout 162 is configured such that when the inner actuator portion 152 is pulled in the direction D', the flexing portion 159 flexes in the D' direction. Referring to FIGS. 5-6, the flexing of the flexing portion 159 and corresponding movement of the sealing portion 154 of the poppet 150 in the D' direction allows the by-pass channel 180 to at least partially form between the sealing portion 154 and the valve seat 172. Fluid A' can flow through the opened by-pass channel and flush the DPT 100.

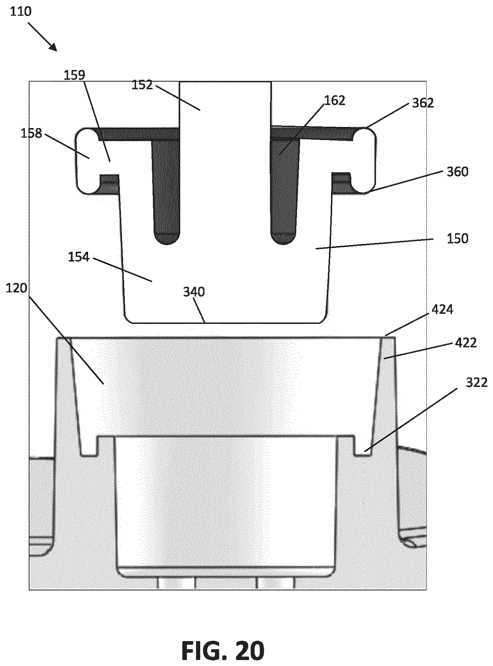

FIG. 20 illustrates an exemplary embodiment of a poppet 150 with an alternate mounting portion 158. With reference to FIG. 20, a portion of a poppet 150 and a housing 120 of a DPT 110 are illustrated. In this exemplary embodiment, the mounting portion 158 is annular with a "dog-bone" cross-sectional shape. This mounting portion 158 includes projections 360, 362 that extend outward axially in opposite directions. The projection 360 of poppet 150 can correspond to a slot 322 located in the housing 120. During manufacturing of the DPT 110, the poppet 150 can be secured to the housing 120. In various embodiments, the projection 362 can fit into slot 322 to form a secure fit between the poppet 150 and the housing 120.

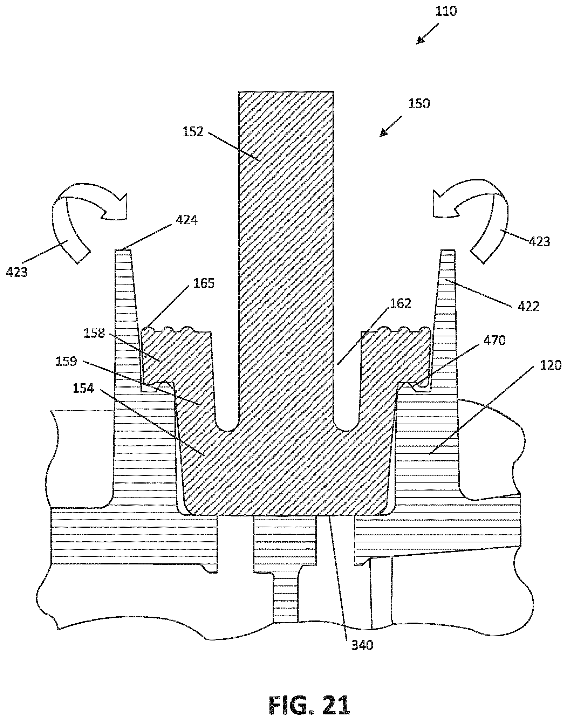

The poppet 150 can be assembled with the housing 120 in a wide variety of different ways. For example, the mounting portion 158 can be attached to the housing 120 with fasteners, by welding, such as ultrasonic welding, with adhesive, by co-molding, by swaging, by securing a cap to the housing 120, etc. FIGS. 21-23 illustrate one of the ways for securing the mounting portion 158 to the housing 120. With reference to FIG. 21, the poppet 150 is placed into the poppet cavity 304 of the housing 120. Before the poppet 150 is installed in the housing, the illustrated housing 120 includes a cylinder 422 that extends around the poppet 150. An open end of the cylinder 422 can be swaged, melted, and/or otherwise pushed and deformed toward and onto the mounting portion 158 of the poppet 150 to secure the poppet to the housing 120 of the DPT 110 as indicated by arrows 423.

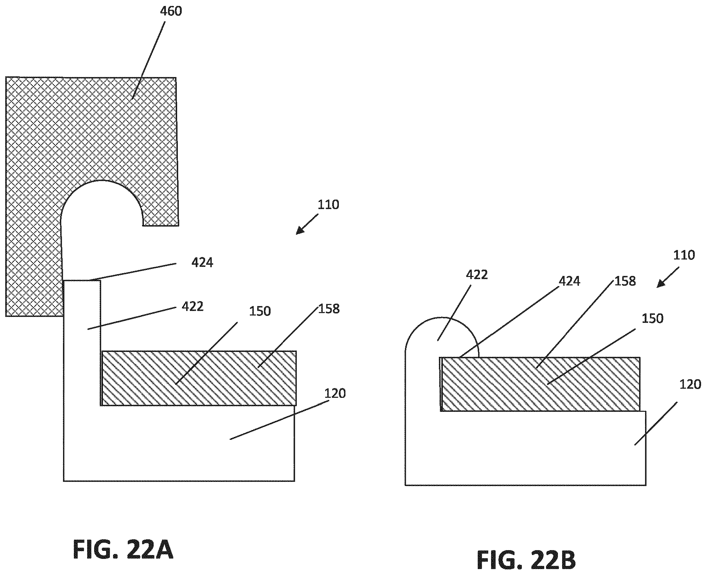

With reference to FIG. 22A, a tool 460 can be used to close the open end of the cylinder 422 onto the mounting portion 158 of the poppet 450. For example, the tool 460 can melt and/or deform the material at the end of the cylinder 422. In various embodiments, and with reference to FIG. 22B, the end of the cylinder 422 is deformed such that the material at the end 424 of the cylinder 422 is pressed against the mounting portion 158 of the poppet 150.

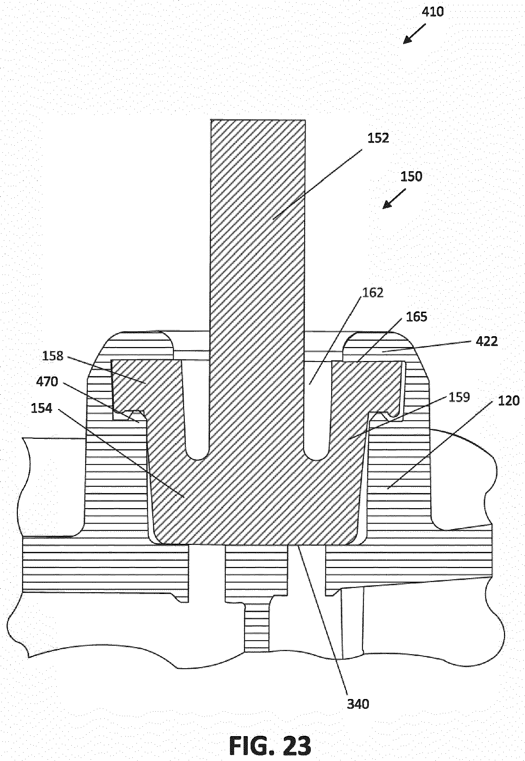

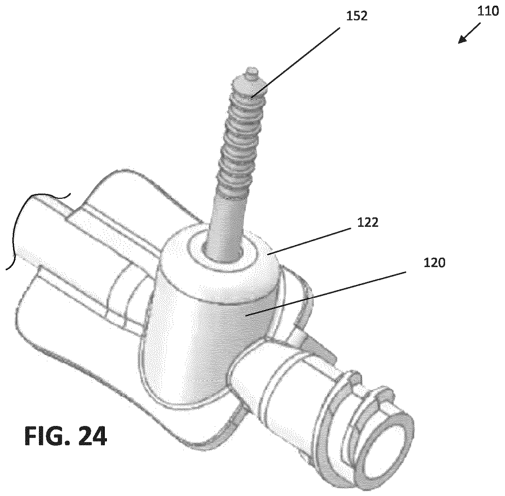

With reference to FIGS. 23-24, the end 423 of the poppet cylinder 422 has been deformed such that the end portion secures the mounting portion 154 of the poppet 150 to the housing 120. In the example illustrated by FIG. 23, an annular projection 470 presses into a bottom surface of the mounting portion 154 to create a first or primary seal between the poppet 150 and the housing 120. The annular projections 165 (See Figure 21) are compressed by the end portion 423 of the poppet cylinder 422 to create a second or secondary seal between the poppet 150 and the housing 120. The compression of the mounting portion 154 between the end 423 of the poppet cylinder 422 and the annular projection 470 also secures the poppet 150 in position relative to the housing 120. FIG. 24 illustrates a perspective view of the DPT 110, with the poppet 150 secured in the cylinder 122 of the housing 120.

The DPT 110 disclosed herein can be used in a wide variety applications. For example, the DPT can have a variety of different types of valves for delivering medication and/or fluids to a patient. Referring to FIG. 25, in one exemplary embodiment a DPT 110 can include a housing 120, a mounting assembly 530, a two-port stopcock assembly 534, and a poppet 150. The stopcock assembly 534 can take a wide variety of different ways. In the example illustrated by FIG. 25, a central axis of the inlet port 535 of the stopcock assembly 534 is co-planar with a central axis of the cylinder 122. The stopcock assembly 534 is connected to the outlet 140 of the of the housing 120. The housing 120 can be coupled with the mounting plate 532 in a wide variety of different ways. For example, the housing 120 can be coupled with the mounting plate 532 by ultrasonic welding, adhesive, fasteners, etc.

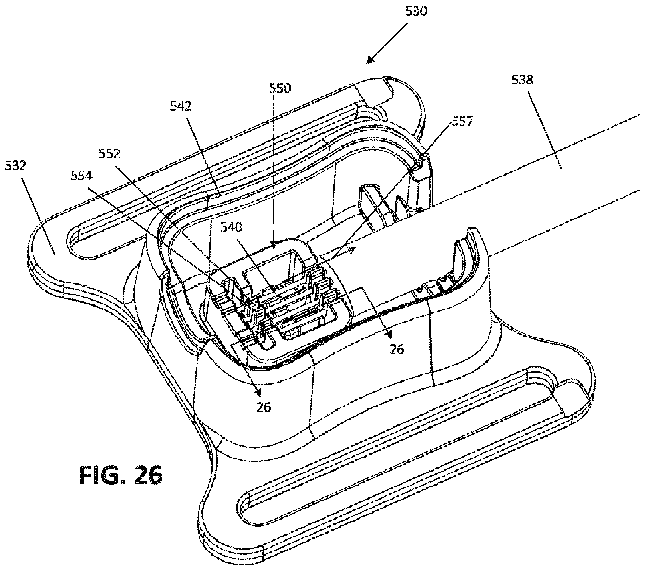

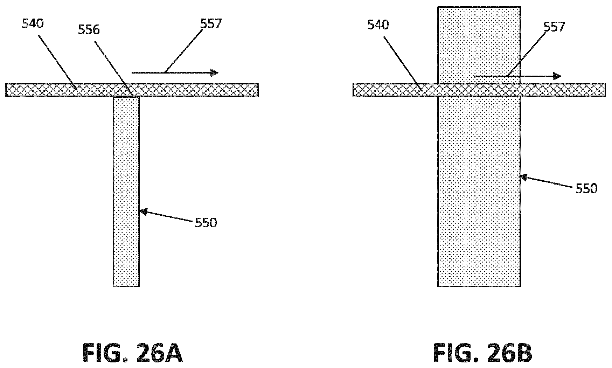

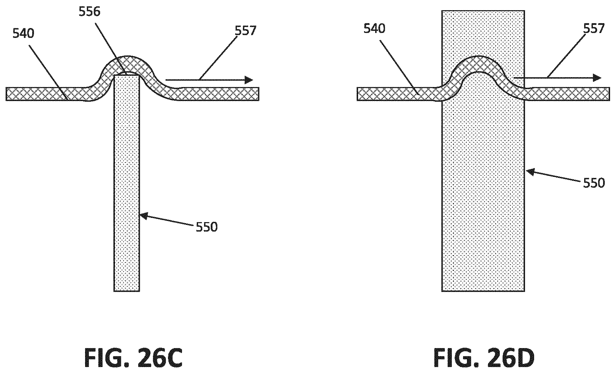

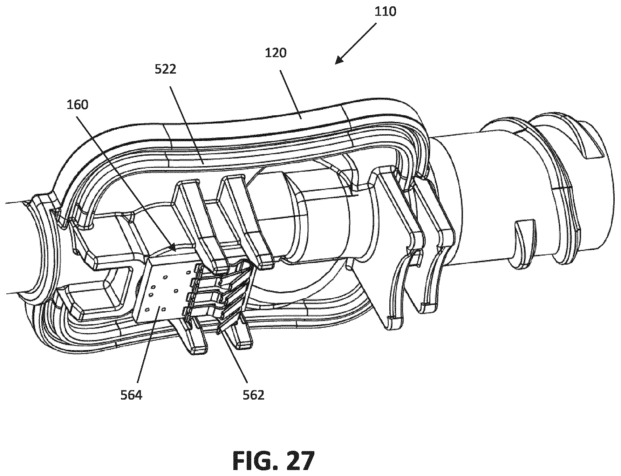

With reference to FIG. 26, the mounting assembly 530 includes a mounting plate 532 and wires ends 540 that are part of a cable 538. The wire ends 540 can optionally be tinned to prevent corroding. Mounting plate 532 can include shaped walls 542 that engage complementary walls 522 in the housing 120 (FIG 27). The shaped walls 542 and the complementary walls 522 of the housing 120 can be connected together in a wide variety of different ways. For example, the walls 542, 522 can be connected together by ultrasonic welding, adhesive, fasteners, etc.

Still referring to FIG. 26, in the illustrated embodiment the mounting plate 532 includes a wire end support portion 550. The wire end support portion 550 holds the wire ends 540 in place in predetermined, spaced apart positions. For example, the spacing and positioning of the wire ends 540 can correspond to terminals 562 of the pressure sensor assembly. The wire support portion 550 can take a wide variety of different forms. Any structure that holds the wire ends in place relative to the mounting plate 532 and maintains the spacing of the wire ends 540 can be used.

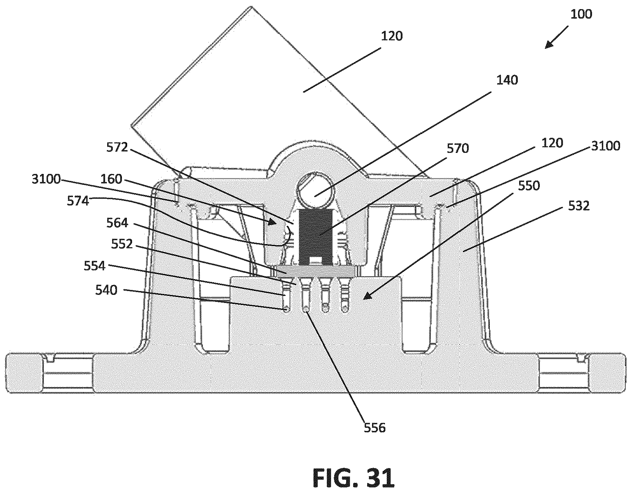

In the example illustrated by FIG. 26, the wire support portion 550 comprises a plurality of columns 552 that are spaced apart by a plurality of channels 554. The channels 554 include a bottom surface 556 that supports the wire ends 540. Referring to FIGS. 26 and 31 the widths of the channels 554 are selected to tightly hold the wire ends 540. FIG. 26A is a cross-sectional view taken along the plane indicated by lines 26-26 in FIG. 26 that illustrates the wire ends 540 resting on the bottom surface 556 of the channels 554.

In one exemplary embodiment, the wire ends 540 are anchored to prevent movement of the wire ends 540 when an axial load 557 is applied. For example, the load 557 can be applied when the cable 538 and/or the individual wires in the cable are pulled. The wire ends 540 can be anchored in a wide variety of different ways. For example, plastic can be molded around the wires, the wires can be bent, a stop, such as metal ring, sphere, etc., can be swaged onto or otherwise attached to the wire ends, and/or the wire ends 540 an be provided with holes, pores, bores, roughened, or otherwise treated to increase friction.

FIGS. 26B-26D illustrate a few examples of anchoring the wire ends 540. These examples are schematically illustrated generally as they would be perceived in a cross-sectional view taken along the plane indicated by lines 26-25 in FIG. 26. The anchoring that is schematically illustrated by FIGS. 26B-26D can be applied to the wire support portion 550, such as to one or more of the sets of columns 552 and/or channels 554, and/or to the wire ends 540. In FIG. 26B, the material, such as plastic, of the columns 552, another portion of the mounting plate 532, and/or a portion of the valve body 120 is melted, molded, and/or otherwise formed around the wire ends 540. In FIG. 26C, the wire end 540 is bent over the bottom surface 556 of the channel 554. In FIG. 26D, the wire end 540 is bent over the bottom surface 556 of the channel 554 and the material, such as plastic, of the columns 552, another portion of the mounting plate 532, and/or a portion of the valve body 120 is melted, molded, and/or otherwise formed around the bent portion of the wire end 540.

In previous DPT assemblies, the terminals associated with the pressure sensor or transducer are soldered to wiring associated with the mounting assembly. This, however, can be time consuming and expensive. In one exemplary embodiment, a terminals 564 of a pressure sensor 160 are electrically coupled to wire ends 540 without soldering. This electrical coupling can be achieved in a wide variety of different ways. For example, the terminals 564 can be pressed into contact with the wire ends 540, can be encased together in plastic, the wire ends 540 can be inserted into terminals 564, and/or the terminals 564 can be inserted into wire ends 540.

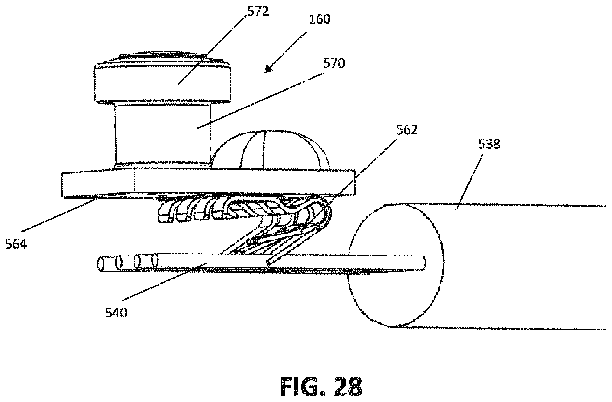

FIGS. 27 and 28 illustrate one exemplary embodiment where the terminals 564 of a pressure sensor 160 are electrically coupled to wire ends 540 without soldering. In this example, the terminals 562 associated with a circuit board 564 of the pressure sensor contact the wire ends 540 of the cable assembly 538. The contact of the wire ends 540 and terminals 562 allows for the signals or readings associated with the pressure sensor 160 to be transferred to a processor, display or other signal reading or interpreting means. With reference to FIGS. 27-28, the terminals 562 are biased against the wire ends 540 and can flex towards and away from the printed circuit board 564 to ensure constant contact with the wire ends 540.

The wire ends 540 and the terminals 562 can be held together as shown in FIG. 28 is a wide variety of different ways. In one exemplary embodiment, assembly of the housing 120 and mounting plate 532 around the pressure sensor 160 and cable 538 holds the wire ends 540 and the terminals 562 against one another.

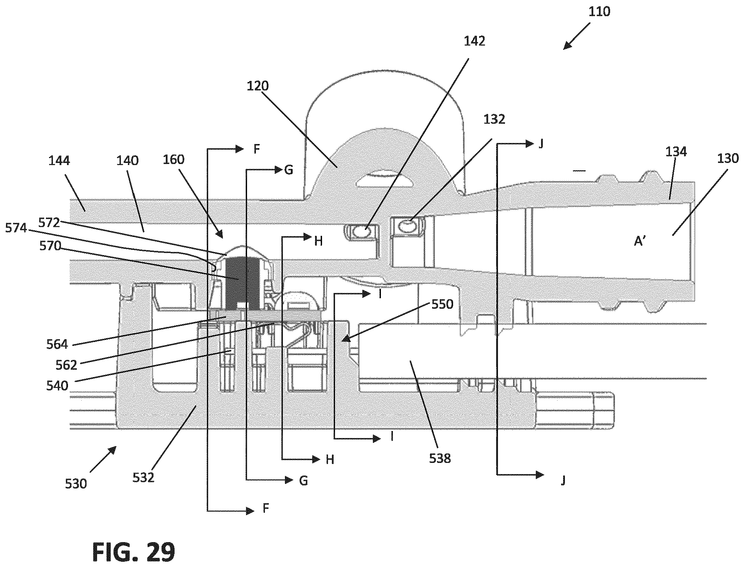

With reference to FIG. 29, a cross section of the DPT 110 of FIG. 25 taken along plane E is illustrated. The housing 120 is coupled with mounting assembly 532 around the pressure sensor 160 and the cable 538. The pressure sensor 160 includes a sensing component 570 that is covered with a seal 572. The seal 572 can be made of comprise one or more of rubber, a synthetic rubber, a synthetic rubber-like material, silicone, or other known sealing material. The sensing component 570 and the seal 572 are held in an opening 574 in the housing 120 that is in communication with the outlet passage 140. In one exemplary embodiment, the seal 572 provides a seal between the pressure sensing component 570 and the opening 574, without requiring any additional components, and places the sensing component 570 is sensing communication with the fluid in the outlet passage 140 (See FIG. 31).

Still referring to FIG. 29, the housing 120 and the mounting assembly 532 clamp against the circuit board 564 to hold the pressure sensor 160 in place. The housing 120 and the mounting assembly 532 also clamp against the cable 538 to hold the cable in place with the wire ends 540 held in place in the wire support portion 550. The walls 542 of the mounting plate 532 (See FIG. 25) are ultrasonically welded, glued, or are otherwise coupled with the walls 522 of the housing 120 (See FIG. 28), such that the wiring 540 is coupled with the terminals 562.

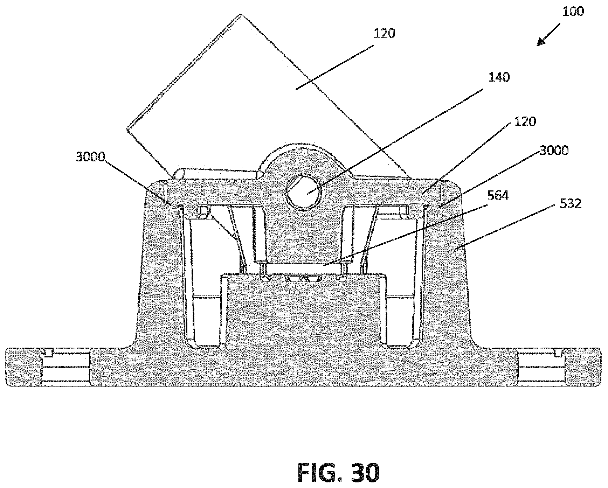

With reference to FIGS. 30-34, cross sections of the DPT 110 of FIG. 29 are illustrated. FIG. 30 illustrates a cross section of the DPT 110 of FIG. 29 along the plane indicated by lines F-F. In FIG. 30 the housing 120 and the mounting plate 532 are connected together at interfaces 3000. The housing 120 and the mounting plate 532 clamp against the circuit board 564 to hold the pressure sensor 160 in place.

FIG. 31 illustrates a cross section of the DPT 110 of FIG. 29 along the plane indicated by lines G-G. In FIG. 31 the housing 120 and the mounting plate 532 are connected together at interfaces 3100. The housing 120 and the mounting plate 532 clamp against the circuit board 564 to hold the pressure sensor 160 in place. This holds the sensing component 570 and the seal 572 in the opening 574 in the housing 120. The sensing component is in communication with the outlet passage 140. The seal 572 provides a seal between the pressure sensing component 570 and the opening 574. The seal 572 can be a separate component that is simply placed over or around the sensing component 570. In one exemplary embodiment, there is no adhesive between the seal 572 and the sensing component 570.

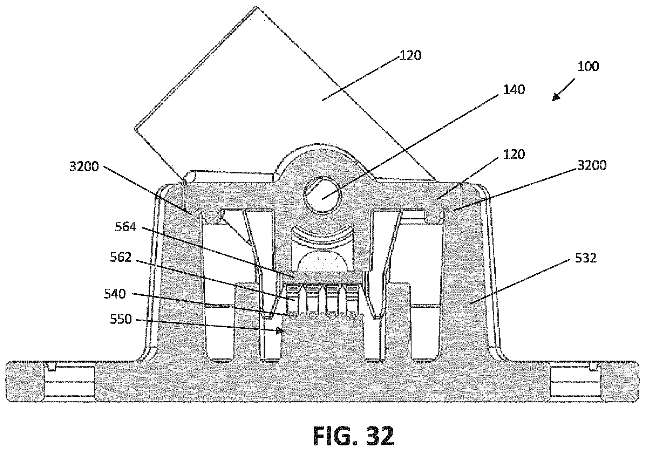

FIG. 32 illustrates a cross section of the DPT 110 of FIG. 29 along the plane indicated by lines H-H. In FIG. 32 the housing 120 and the mounting plate 532 are connected together at interfaces 3200. The assembly of the housing 120 and mounting plate 532 around the pressure sensor 160 and cable 538 holds the wire ends 540 and the terminals 562 against one another.

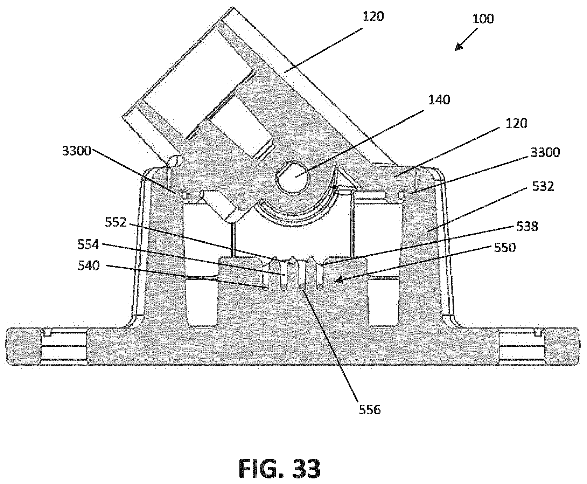

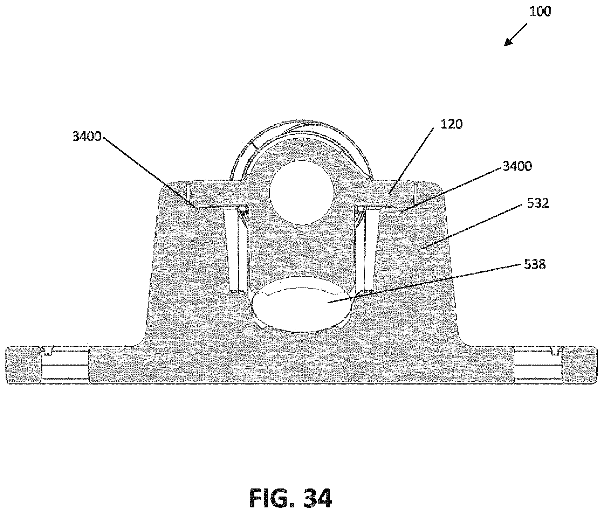

FIG. 33 illustrates a cross section of the DPT 110 of FIG. 29 along the plane I. FIG. 34 illustrates a cross section of the DPT 510 of FIG. 29 along the plane J. In FIG. 33 the housing 120 and the mounting plate 532 are connected together at interfaces 3300. The assembly of the housing 120 and mounting plate 532 around the cable 538 holds the wire ends 540 in the channels 554.

FIG. 34 illustrates a cross section of the DPT 110 of FIG. 29 along the plane indicated by lines I-I. In FIG. 34 the housing 120 and the mounting plate 532 are connected together at interfaces 3400. The assembly of the housing 120 and mounting plate 532 clamps around the cable 538 to hold the cable in place and provide a strain relief for the wire ends 540.

Disclosed herein is a method of flushing a disposable pressure transducer can include projecting a fluid A' through a first flow path B' comprising an inlet port 132, a flow restrictor 170, and an outlet port 142. In various embodiments, the flow restrictor 170 is disposed on a valve seat 172 of a housing 120 of the pressure transducer 110. In various embodiments, the method can include decoupling a poppet 150 from the valve seat 172 of the housing 120, allowing the fluid A' to travel through a second flow path B' comprising the inlet port 132, a by-pass channel 180, and the outlet port 142. In various embodiments, the decoupling the poppet 150 comprises exerting a force on poppet 150 in a direction D away from the housing 120. In various embodiments, the method can include coupling the poppet 150 with the valve seat 1172 of the housing 120 to close the by-pass channel 180. In various embodiments, the first flow path B has a smaller flow rate than the second flow rate B'. In various embodiments, the first flow path B provides a flow rate between about 1 cc/hr to about 10 cc/hr. In various embodiments, the second flow path B' provides a flow rate between about 5 cc/min to about 250 cc/min.

While various inventive aspects, concepts and features of the disclosures may be described and illustrated herein as embodied in combination in the exemplary embodiments, these various aspects, concepts, and features may be used in many alternative embodiments, either individually or in various combinations and sub-combinations thereof. Unless expressly excluded herein all such combinations and sub-combinations are intended to be within the scope of the present application. Still further, while various alternative embodiments as to the various aspects, concepts, and features of the disclosures-such as alternative materials, structures, configurations, methods, devices, and components, alternatives as to form, fit, and function, and so on-may be described herein, such descriptions are not intended to be a complete or exhaustive list of available alternative embodiments. Those skilled in the art may readily adopt one or more of the aspects, concepts, or features into additional embodiments and uses within the scope of the present application even if such embodiments are not expressly disclosed herein.

Additionally, even though some features, concepts, or aspects of the disclosures may be described herein as being a preferred arrangement or method, such description is not intended to suggest that such feature is required or necessary unless expressly so stated. Still further, exemplary or representative values and ranges may be included to assist in understanding the present application, however, such values and ranges are not to be construed in a limiting sense and are intended to be critical values or ranges only if so expressly stated.

Moreover, while various aspects, features and concepts may be expressly identified herein as being inventive or forming part of a disclosure, such identification is not intended to be exclusive, but rather there may be aspects, concepts, and features that are fully described herein without being expressly identified as such or as part of a specific disclosure, the disclosures instead being set forth in the appended claims. Descriptions of exemplary methods or processes are not limited to inclusion of all steps as being required in all cases, nor is the order that the steps are presented to be construed as required or necessary unless expressly so stated. The words used in the claims have their full ordinary meanings and are not limited in any way by the description of the embodiments in the specification.

## Claims

1. A pressure transducer (110) comprising:
a housing (120) comprising an inlet port (132), an outlet port (142) and a valve seat (172);
a poppet (150) coupled with the housing (120), wherein the poppet (150) comprises a sealing portion (154); and
a pressure transducer;
wherein a flow restrictor (170) is defined by the valve seat (172) between the inlet port (132) and the outlet port (142);
wherein the flow restrictor (170) comprises a flow restrictor channel (174) extending into the valve seat (172);
wherein the poppet (150) is coupled with the valve seat (172);
wherein the housing (120) includes either or both of:
a) a ramp (176) between the flow restrictor channel (174) and the inlet port (132) wherein the ramp (176) between the flow restrictor channel (174) and the inlet port (132) is configured to provide extra space to reduce the risk of a blockage of an entrance of the flow restrictor channel (174) caused by load that presses the sealing portion (154) of the poppet (150) into the inlet port (132); and
b) a ramp (176) between the flow restrictor channel (174) and the outlet port (142), wherein the ramp (176) between the flow restrictor channel (174) and the outlet port (142) is configured to provide extra space to reduce the risk of a blockage of an exit of the flow restrictor channel (174) caused by load that presses the sealing portion (154) of the poppet (150) into the outlet port (142); and
wherein the pressure transducer is configured to allow a fluid to flow within a first flow path comprising the inlet port (132), the flow restrictor channel (174), the outlet port (142) and the ramp or ramps (176) in response to the sealing portion (154) of the poppet (150) being closed against the valve seat (172).

2. The pressure transducer of claim 1, wherein the valve seat is integrally formed with the housing.

3. The pressure transducer of claim 1 or claim 2, wherein the flow rate of the fluid within the first flow path is between about 1 cc/hr and about 10 cc/hr.

4. The pressure transducer of any preceding claim, further comprising a by-pass channel (180) between the poppet and the valve seat of the housing.

5. The pressure transducer of claim 4, wherein a fluid flows through a second flow path comprising the inlet port, the by-pass channel, and the outlet port in response to the poppet being decoupled from the valve seat of the housing.

6. The pressure transducer of claim 5, wherein the flow rate of the fluid within the second flow path is between about 5 cc/min to about 250 cc/min.

7. The pressure transducer of claim 1, wherein the flow restrictor channel comprises:
a) a width between 0.0127mm (0.0005 inches) and 0.2032 (0.0080 inches); or
b) a depth between 0.0127mm (0.0005 inches) and 0.2032 (0.0080 inches).

8. The pressure transducer of any preceding claim, wherein a depth of the channel is greater than a width of the channel.

9. The pressure transducer of any preceding claim, wherein the flow restrictor channel comprises a length that is at least twice the distance between the inlet port and the outlet port.

10. The pressure transducer of any preceding claim, wherein the flow restrictor channel comprises a rounded, rectangular, or trapezoidal shape in cross section.

11. A method of flushing a pressure transducer (110), comprising:
projecting a fluid (A') through a first flow path (B) comprising an inlet port (132), a flow restrictor (170), an outlet port (142) and a ramp (176) or ramps (176), wherein the flow restrictor (170) is disposed on a valve seat (172) of a housing (120) of the pressure transducer (110), wherein the flow restrictor (170) comprises a flow restrictor channel (174) extending into the valve seat (172) of the housing (120) ; and
decoupling a sealing portion (154) of a poppet (150) from the valve seat (172) of the housing (120), allowing the fluid (A') to travel through a second flow path (B') comprising the inlet port (132), a by-pass channel (180), and the outlet port (142);
wherein the housing (120) includes either or both of:
a) a ramp (176) between the flow restrictor channel (174) and the inlet port (132), wherein the ramp (176) between the flow restrictor channel (174) and the inlet port (132) is configured to provide extra space to reduce the risk of a blockage of an entrance of the flow restrictor channel (174) caused by load that presses the sealing portion (154) of the poppet into the inlet port (132); and
b) a ramp (176) between the flow restrictor channel (174) and the outlet port (132)wherein the ramp (176) between the flow restrictor channel (174) and the outlet port (142) is configured to provide extra space to reduce the risk of a blockage of an exit of the flow restrictor channel (174) caused by load that presses the sealing portion (154) of the poppet into the outlet port (142).

12. The method of claim 11, wherein the decoupling the poppet comprises exerting a force on the poppet in a direction away from the valve seat.

13. The method of claim 11 or claim 12, further comprising coupling the poppet with the valve seat of the housing to close the by-pass channel.

14. The method of any of claims 11 to 13, wherein the first flow path has a smaller flow rate than the second flow rate.

15. The method of claim 14, wherein the first flow path comprises a flow rate between about 1 cc/hr to about 10 cc/hr and wherein the second flow path comprises a flow rate between about 5 cc/min to about 250 cc/min.

## Patentansprüche

1. Druckwandler (110), umfassend:
ein Gehäuse (120), das eine Einlassöffnung (132), eine Auslassöffnung (142) und einen Ventilsitz (172) umfasst;
ein Tellerventil (150), das mit dem Gehäuse (120) gekoppelt ist, wobei das Tellerventil (150) einen Dichtabschnitt (154) umfasst; und
einen Druckwandler;
wobei ein Durchflussbegrenzer (170) durch den Ventilsitz (172) zwischen der Einlassöffnung (132) und der Auslassöffnung (142) definiert ist;
wobei der Durchflussbegrenzer (170) einen Durchflussbegrenzerkanal (174) umfasst, der sich in den Ventilsitz (172) erstreckt;
wobei das Tellerventil (150) mit dem Ventilsitz (172) gekoppelt ist;
wobei das Gehäuse (120) eins oder beide aufweist von:
a) einer Rampe (176) zwischen dem Durchflussbegrenzerkanal (174) und der Einlassöffnung (132), wobei die Rampe (176) zwischen dem Durchflussbegrenzerkanal (174) und der Einlassöffnung (132) dazu ausgelegt ist, zusätzlichen Raum bereitzustellen, um das Risiko einer Blockierung eines Eingangs des Durchflussbegrenzerkanals (174) zu verringern, die durch Last, die den Dichtabschnitt (154) des Tellerventils (150) in die Einlassöffnung (132) drückt, hervorgerufen wird; und
b) einer Rampe (176) zwischen dem Durchflussbegrenzerkanal (174) und der Auslassöffnung (142), wobei die Rampe (176) zwischen dem Durchflussbegrenzerkanal (174) und der Auslassöffnung (142) dazu ausgelegt ist, zusätzlichen Raum bereitzustellen, um das Risiko einer Blockierung eines Ausgangs des Durchflussbegrenzerkanals (174) zu verringern, die durch Last, die den Dichtabschnitt (154) des Tellerventils (150) in die Auslassöffnung (142) drückt, hervorgerufen wird; und
wobei der Druckwandler dazu ausgelegt ist, zu ermöglichen, dass ein Fluid in einem ersten Fließweg, der die Einlassöffnung (132), den Durchflussbegrenzerkanal (174), die Auslassöffnung (142) und die Rampe oder die Rampen (176) umfasst, in Reaktion darauf fließen kann, dass der Dichtabschnitt (154) des Tellerventils (150) gegen den Ventilsitz (172) geschlossen wird.

2. Druckwandler nach Anspruch 1, wobei der Ventilsitz einstückig mit dem Gehäuse gebildet ist.

3. Druckwandler nach Anspruch 1 oder 2, wobei die Durchflussmenge des Fluids in dem ersten Fließweg zwischen etwa 1 cm³/h und etwa 10 cm³/h beträgt.

4. Druckwandler nach einem vorhergehenden Anspruch, ferner umfassend einen Umgehungskanal (180) zwischen dem Tellerventil und dem Ventilsitz des Gehäuses.

5. Druckwandler nach Anspruch 4, wobei ein Fluid durch einen zweiten Fließweg, der die Einlassöffnung, den Bypasskanal und die Auslassöffnung umfasst, in Reaktion darauf fließt, dass das Tellerventil von dem Ventilsitz des Gehäuses entkoppelt wird.

6. Druckwandler nach Anspruch 5, wobei die Durchflussmenge des Fluids in dem zweiten Fließweg zwischen etwa 5 cm³/Min. und etwa 250 cm³/Min. beträgt.

7. Druckwandler nach Anspruch 1, wobei der Durchflussbegrenzerkanal umfasst:
a) eine Breite zwischen 0,0127 mm (0,0005 Zoll) und 0,2032 mm (0,0080 Zoll); oder
b) eine Tiefe zwischen 0,0127 mm (0,0005 Zoll) und 0,2032 mm (0,0080 Zoll).

8. Druckwandler nach einem vorhergehenden Anspruch, wobei eine Tiefe des Kanals größer ist als eine Breite des Kanals.

9. Druckwandler nach einem vorhergehenden Anspruch, wobei der Durchflussbegrenzerkanal eine Länge umfasst, die mindestens zweimal so groß ist wie der Abstand zwischen der Einlassöffnung und der Auslassöffnung.

10. Druckwandler nach einem vorhergehenden Anspruch, wobei der Durchflussbegrenzerkanal einen abgerundeten, rechteckigen oder trapezförmigen Querschnitt umfasst.

11. Verfahren zur Spülung eines Druckwandlers (110), umfassend:
Durchleiten eines Fluids (A') durch einen ersten Fließweg (B), der eine Einlassöffnung (132), einen Durchflussbegrenzer (170), eine Auslassöffnung (142) und eine Rampe (176) oder Rampen (176) umfasst, wobei der Durchflussbegrenzer (170) an einem Ventilsitz (172) eines Gehäuses (120) des Druckwandlers (110) angeordnet ist, wobei der Durchflussbegrenzer (170) einen Durchflussbegrenzerkanal (174) umfasst, der sich in den Ventilsitz (172) des Gehäuses (120) erstreckt; und
Entkoppeln eines Dichtabschnitts (154) eines Tellerventils (150) von dem Ventilsitz (172) des Gehäuses (120), was ermöglicht, dass das Fluid (A') durch einen zweiten Fließweg (B') wandert, der die Einlassöffnung (132), einen Bypasskanal (180) und die Auslassöffnung (142) umfasst;
wobei das Gehäuse (120) eins oder beide aufweist von:
a) einer Rampe (176) zwischen dem Durchflussbegrenzerkanal (174) und der Einlassöffnung (132), wobei die Rampe (176) zwischen dem Durchflussbegrenzerkanal (174) und der Einlassöffnung (132) dazu ausgelegt ist, zusätzlichen Raum bereitzustellen, um das Risiko einer Blockierung eines Eingangs des Durchflussbegrenzerkanals (174) zu verringern, die durch Last, die den Dichtabschnitt (154) des Tellerventils in die Einlassöffnung (132) drückt, hervorgerufen wird; und
b) einer Rampe (176) zwischen dem Durchflussbegrenzerkanal (174) und der Auslassöffnung (132), wobei die Rampe (176) zwischen dem Durchflussbegrenzerkanal (174) und der Auslassöffnung (142) dazu ausgelegt ist, zusätzlichen Raum bereitzustellen, um das Risiko einer Blockierung eines Ausgangs des Durchflussbegrenzerkanals (174) zu verringern, die durch Last, die den Dichtabschnitt (154) des Tellerventils in die Auslassöffnung (142) drückt, hervorgerufen wird.

12. Verfahren nach Anspruch 11, wobei das Entkoppeln des Tellerventils das Ausüben einer Kraft auf das Tellerventil in eine Richtung weg vom Ventilsitz umfasst.

13. Verfahren nach Anspruch 11 oder Anspruch 12, ferner umfassend das Koppeln des Tellerventils mit dem Ventilsitz des Gehäuses, um den Bypasskanal zu verschließen.

14. Verfahren nach einem der Ansprüche 11 bis 13, wobei der erste Fließweg eine geringere Durchflussmenge als die zweite Durchflussmenge hat.

15. Verfahren nach Anspruch 14, wobei der erste Fließweg eine Durchflussmenge zwischen etwa 1 cm³/h bis etwa 10 cm³/h umfasst und wobei der zweite Fließweg eine Durchflussmenge zwischen etwa 5 cm³/Min. bis etwa 250 cm³/Min. umfasst.

## Revendications

1. Transducteur de pression (110) comprenant :
un boîtier (120) comprenant un orifice d'entrée (132), un orifice de sortie (142) et un siège de soupape (172) ;
un clapet (150) couplé au boîtier (120), le clapet (150) comprenant une partie d'étanchéité (154) ; et
un transducteur de pression ;
un réducteur de débit (170) étant défini par le siège de soupape (172) entre l'orifice d'entrée (132) et l'orifice de sortie (142) ;
le réducteur de débit (170) comprenant un canal de réduction de débit (174) s'étendant dans le siège de soupape (172) ;
le clapet (150) étant couplé au siège de soupape (172) ;
le boîtier (120) comprenant l'un ou l'autre ou les deux éléments suivants :
a) une rampe (176) entre le canal de réduction de débit (174) et l'orifice d'entrée (132), la rampe (176) entre le canal de réduction de débit (174) et l'orifice d'entrée (132) étant configurée pour fournir un espace supplémentaire afin de réduire le risque de blocage d'une entrée du canal de réduction de débit (174) causé par une charge qui presse la partie d'étanchéité (154) du clapet (150) dans l'orifice d'entrée (132) ; et
b) une rampe (176) entre le canal de réduction de débit (174) et l'orifice de sortie (142), la rampe (176) entre le canal de réduction de débit (174) et l'orifice de sortie (142) étant configurée pour fournir un espace supplémentaire afin de réduire le risque de blocage d'une sortie du canal de réduction de débit (174) causé par une charge qui presse la partie d'étanchéité (154) du clapet (150) dans l'orifice de sortie (142) ; et
le transducteur de pression étant configuré pour permettre à un fluide de s'écouler dans un premier chemin d'écoulement comprenant l'orifice d'entrée (132), le canal de réduction de débit (174), l'orifice de sortie (142) et la ou les rampes (176) en réponse à la fermeture de la partie d'étanchéité (154) du clapet (150) contre le siège de soupape (172).

2. Transducteur de pression selon la revendication 1, le siège de soupape étant formé d'un seul tenant avec le boîtier.

3. Transducteur de pression selon la revendication 1 ou la revendication 2, le débit du fluide à l'intérieur du premier chemin d'écoulement étant compris entre environ 1 cm³/h et environ 10 cm³/h.

4. Transducteur de pression selon l'une quelconque des revendications précédentes, comprenant en outre un canal de dérivation (180) entre le clapet et le siège de soupape du boîtier.

5. Transducteur de pression selon la revendication 4, un fluide s'écoulant à travers un second chemin d'écoulement comprenant l'orifice d'entrée, le canal de dérivation et l'orifice de sortie en réponse au découplage du clapet du siège de soupape du boîtier.

6. Transducteur de pression selon la revendication 5, le débit du fluide dans le second chemin d'écoulement étant compris entre environ 5 cm³/min et environ 250 cm³/min.

7. Transducteur de pression selon la revendication 1, le canal de réduction de débit comprenant :
a) une largeur comprise entre 0,0127 mm (0,0005 pouce) et 0,2032 (0,0080 pouce) ; ou
b) une profondeur comprise entre 0,0127 mm (0,0005 pouce) et 0,2032 (0,0080 pouce).

8. Transducteur de pression selon l'une quelconque des revendications précédentes, une profondeur du canal étant supérieure à une largeur du canal.

9. Transducteur de pression selon l'une quelconque des revendications précédentes, le canal de réduction de débit comprenant une longueur qui est au moins deux fois la distance entre l'orifice d'entrée et l'orifice de sortie.

10. Transducteur de pression selon l'une quelconque des revendications précédentes, le canal de réduction de débit comprenant une forme arrondie, rectangulaire ou trapézoïdale en coupe transversale.

11. Procédé de rinçage d'un transducteur de pression (110), comprenant :
la projection d'un fluide (A') à travers un premier trajet d'écoulement (B) comprenant un orifice d'entrée (132), un réducteur de débit (170), un orifice de sortie (142) et une rampe (176) ou des rampes (176), le réducteur de débit (170) étant disposé sur un siège de soupape (172) d'un boîtier (120) du transducteur de pression (110), le réducteur de débit (170) comprenant un canal de réduction de débit (174) s'étendant dans le siège de soupape (172) du boîtier (120) ; et
le découplage d'une partie d'étanchéité (154) d'un clapet (150) du siège de soupape (172) du boîtier (120), permettant au fluide (A') de circuler à travers un second chemin d'écoulement (B') comprenant l'orifice d'entrée (132), un canal de dérivation (180) et l'orifice de sortie (142) ;
le boîtier (120) comprenant l'un ou l'autre ou les deux éléments suivants :
a) une rampe (176) entre le canal de réduction de débit (174) et l'orifice d'entrée (132), la rampe (176) entre le canal de réduction de débit (174) et l'orifice d'entrée (132) étant configurée pour fournir un espace supplémentaire afin de réduire le risque de blocage d'une entrée du canal de réduction de débit (174) causé par une charge qui presse la partie d'étanchéité (154) du clapet dans l'orifice d'entrée (132) ; et
b) une rampe (176) entre le canal de réduction de débit (174) et l'orifice de sortie (132), la rampe (176) entre le canal de réduction de débit (174) et l'orifice de sortie (142) étant configurée pour fournir un espace supplémentaire afin de réduire le risque de blocage d'une sortie du canal de réduction de débit (174) causé par une charge qui presse la partie d'étanchéité (154) du clapet dans l'orifice de sortie (142).

12. Procédé selon la revendication 11, le découplage du clapet comprenant l'application d'une force sur le clapet dans une direction s'éloignant du siège de soupape.

13. Procédé selon la revendication 11 ou la revendication 12, comprenant en outre le couplage du clapet avec le siège de soupape du boîtier pour fermer le canal de dérivation.

14. Procédé selon l'une quelconque des revendications 11 à 13, le premier trajet d'écoulement ayant un débit inférieur au second débit.

15. Procédé selon la revendication 14, le premier trajet d'écoulement comprenant un débit compris entre environ 1 cm³/h et environ 10 cm³/h et le second trajet d'écoulement comprenant un débit compris entre environ 5 cm³/min et environ 250 cm³/min.
